(19) Europäisches Patentamt
European Patent Office
Office européen des brevets

(11) Veröffentlichungsnummer: **0 114 374**
**B1**

(12) # EUROPÄISCHE PATENTSCHRIFT

(45) Veröffentlichungstag der Patentschrift: **23.07.86**

(21) Anmeldenummer: **83112952.3**

(22) Anmeldetag: **22.12.83**

(51) Int. Cl.⁴: **C 07 D 311/58**, C 07 D 311/60, C 07 D 311/96, C 07 D 407/12, A 61 K 31/35, A 61 K 31/36

(54) **Substituierte 4-Aminomethylenchromane bzw. -chromene, Verfahren zu ihrer Herstellung sowie ihre Verwendung in Arzneimitteln.**

(30) Priorität: **03.01.83 DE 3300004**

(43) Veröffentlichungstag der Anmeldung: **01.08.84 Patentblatt 84/31**

(45) Bekanntmachung des Hinweises auf die Patenterteilung: **23.07.86 Patentblatt 86/30**

(84) Benannte Vertragsstaaten: **AT BE CH DE FR GB IT LI NL SE**

(56) Entgegenhaltungen: **GB-A-2 089 348**

(73) Patentinhaber: **BAYER AG, Konzernverwaltung RP Patentabteilung, D-5090 Leverkusen 1 Bayerwerk (DE)**

(72) Erfinder: **Widdig, Arno, Dr., Eifgenstrasse 8, D-5068 Odenthal (DE)**
Erfinder: **Kabbe, Hans- Joachim, Dr., Walter- Flex-Strasse 16, D-5090 Leverkusen (DE)**
Erfinder: **Knorr, Andreas, Dr., Pahlkestrasse 15, D-5600 Wuppertal 1 (DE)**
Erfinder: **Benz, Ulrich, Gustav- Poensgenstrasse 29, D-4000 Duesseldorf (DE)**

EP 0 114 374 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft substituierte 4-Amino-methylenchromane und -chromene, mehrere Verfahren zu ihrer Herstellung sowei ihre Verwendung in Arzneimitteln, insbesondere in kreislaufbeeinflussenden Mitteln.

Aus GB—A—2089348 sind bereits Benzopyranderivate mit ungesättigten Substituenten beschrieben, die eine antimykotische Wirkung besitzen. Hinweise auf die erfindungsgemäßen Verbindungen der Formel I und ihre kreislaufbeeinflussende Wirkung sind dem Stand der Technik nicht zu entnehmen.

Die neuen Verbindungen können durch folgende Formel (I) wiedergegeben werden:

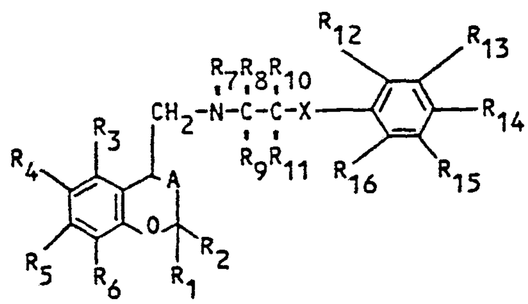

(I)

in der

—A— eine Einfachbindung oder eine Doppelbindung darstellt,

$R_1$ und $R_2$ gleich oder verschieden sind und für Wasserstoff, $C_1$—$C_6$-Alkyl, $C_5$—$C_7$-Cycloakyl, Pehnyl, das gegebenenfalls durch $C_1$—$C_4$-Alkyl, Halogen und/oder $C_1$—$C_4$-Alkoxy substituiert sein kann, sowie für $C_7$—$C_9$-Aralkyl, dessen Arylrest gegebenen falls durch $C_1$—$C_4$-Alkyl, Halogen und/oder $C_1$—$C_4$-Alkoxy substituiert ist stehen,

oder $R_1$ und $R_2$ zusammen mit dem eingeschlossenen C-Atom des Chromanringes einen 4—7 gliedrigen carbocyclischen Ring bilden,

$R_3$ bis $R_6$ gleich oder verschieden sind und für Wasserstoff, Hydroxy, Halogen, $C_1$—$C_6$-Alkyl, $C_5$—$C_7$-Cycloalkyl, Phenyl, das gegebenenfalls durch $C_1$—$C_4$-Alkyl, Halogen und/oder $C_1$—$C_4$-Alkoxy substituiert ist, sowie für $C_7$—$C_9$-Aralkyl, dessen Arylrest gegebenenfalls durch $C_1$—$C_4$-Alkyl, Halogen und/oder $C_1$—$C_4$-Alkyloxy substituiert ist, oder für $C_1$—$C_4$-Alkoxy stehen,

$R_7$, $R_8$, $R_9$, $R_{10}$ gleich oder verschieden sind und für Wasserstoff oder $C_1$—$C_6$-Alkyl stehen,

X für eine Einfachbindung, Methylen das gegebenenfalls eine- oder zweifach durch $C_1$—$c_4$-Alkyl oder für Sauerstoff oder —$NR_{17}$ substituiert ist, steht, wobei $R_{17}$ Wasserstoff oder $C_1$—$C_4$-Alkyl darstellt oder $R_{17}$ zusammen mit $R_7$ einen $C_2$-Alkylenringschluß bildet, und

$R_{12}$, $R_{13}$, $R_{14}$, $R_{15}$, $R_{16}$ gleich oder verschieden sind und für Wasserstoff, Hydroxy, Halogen, $C_1$—$C_6$-Alkyl, $C_5$— oder $C_6$-cycloalkyl, Benzyl, $C_1$—$C_4$-Alkoxy, Trifluormethyl bedeuten, wobei $R_{12}$ und $R_{13}$ oder $R_{13}$ und $R_{14}$ zusammen jeweils eine $C_1$—$C_3$-alkylendioxygruppe oder eine —CH=CH—CH=CH-Gruppe bilden können,

sowie ihre pharmazeutisch unbedenklichen Säureadditions-salze.

Als Säuren zur Herstellung der Salze seien beispielsweise genannt: Schwefelsäure, Salzsäure, organische Carbonsäuren wie Äpfelsäure, Citronensäure, Fumarsäure, Essigsäure oder organische Sulfonsäuren wie Naphthalin-1,5-di-sulfonsäure.

Die Salze der Verbindungen der Formel (I) können in einfacher Weise nach üblichen Salzbildungsmethoden, z.B. durch Lösen der Base und Hinzufügen der Säure erhalten werden und in bekannter Weise, z.B. durch Abfiltrieren, isoliert und gegebenenfalls gereinigt werden.

Bevorzugt stehen in Formel (I):

$R_1$ und $R_2$ gleich oder verschieden sind und für Wasserstoff, $C_1$—$C_6$-Alkyl, $C_5$—$C_7$-Cycloalkyl, Phenyl, das gegebenefalls (2-fach, insbesondere 1-aach) durch $C_1$—$C_4$-Alkyl, Halogen (Chlor, Brom) und/oder $C_1$—$C_4$-Alkoxy substituiert sein kann, sowie für $C_7$—$C_9$-Aralkyl, dessen Arylrest gegebenenfalls (1-2-fach, insbesondere 1-fach) durch $C_1$—$C_4$-Alkyl, Halogen (Chlor, Brom) und/oder $C_1$—$C_4$-Alkoxy substituiert ist, oder $R_1$ und $R_2$ bilden zusammen mit dem eingeschlossenen C-Atom des Chromanringes einen 4 bis 7-gliedrigen carbocyclischen Ring;

$R_3$ bis $R_6$ gleich oder verschieden sind und für Wasserstoff, Hydroxy, Halogen, (Chlor, Brom), $C_1$—$C_6$-Alkyl, $C_5$—$C_7$-Cycloalkyl, Phenyl, das gegebenenfalls (1 bis 2-fach insbesondere 1-fach) durch $C_1$—$C_4$-Alkyl, Halogen (Chlor, Brom) und/oder $C_1$—$C_4$-Alkoxy substituiert ist, sowie fur $C_7$—$C_9$-Aralkyl, dessen Arylrest gegebenenfalls (1 bis 2-fach, insbesondere 1-fach) durch $C_1$—$C_4$-Alkyl, Halogen (Chlor, brom) und/oder $C_1$—$C_4$-Alkoxy substituiert ist, ode für $C_1$—$C_4$-Alkoxy.

$R_7$, $R_8$; $R_9$, $R_{10}$, $R_{11}$ gleich oder verschieden sind und für Wasserstoff oder $C_1$—$C_6$-Alkyl

X für eine Einfachbindung, Methylen das gegebenenfalls ein- oder zweifash durch $C_1$—$C_4$-Alkyl, Sauerstoff oder —$NR_{17}$ substituiert ist, wobei $R_{17}$ Wasserstoff oder $C_1$—$C_4$ Alkyl darstellt oder $R_{17}$ zusammen mit $R_7$ einen $C_2$-Alkylenringschluß bildet, und

$R_{12}$, $R_{13}$, $R_{14}$, $R_{15}$, $R_{16}$ gleich oder verschieden sind und für Wasserstoff, Hydroxy, Halogen (Chlor, Brom Fluor), $C_1$—$C_6$-Alkyl, $C_5$ oder $C_6$ Cycloalkyl, Benzyl, $C_1$—$C_4$-Alkoxy, Trifluormethyl bedeuten, wobei $R_{12}$ und

$R_{13}$ oder $R_{13}$ und $R_{14}$ zusammen jeweils eine $C_1$—$C_3$-Alkylendioxygruppe oder eine —CH=CH—CH=CH-Gruppe bilden können.

Besonders bevorzugt sind Verbindungen der Formel (I), in welcher

$R_1$ und $R_2$ gleich oder verschieden sind und Wasserstoff, $C_1$—$C_4$-Alkyl oder zusammen mit dem von ihnen eingeschlossenen Kohlenstoffatom einen carbocyclischen $C_5$ oder $C_6$ Ring bilden, darstellen,

$R_3$ bis $R_6$ gleich oder verschieden sind und Wasserstoff, Hydroxy, $C_1$—$C_4$-Alkyl, $C_1$—$C_4$-Alkoxy oder Chlor bedeuten,

$R_7$ bis $R_{11}$ gfleich oder verschieden sind und Wasserstoff oder $C_1$—$C_4$-Alkyl darstellen,

X eine Einfachbindung, Sauerstoff, Methylen oder —$NR_{17}$ darstellt, wobei $R_{17}$ Wasserstoff oder $C_1$—$C_3$-Alkyl bedeutet, sowie $R_{17}$ zusammen mit $R_7$ einen Ethylenringschluß bildet, sowie $R_{17}$ zusammen mit $R_7$ einen Ethylenringschluß bildet,

$R_{12}$ bis $R_{16}$ gleich oder verschieden sind und Wasserstoff, Chlor, Cyclohexyl, $C_1$—$C_4$-Alkyl, $C_1$—$C_3$-Alkoxy, Trifluormethyl bedeuten, wobei $R_{12}$ und $R_{13}$ oder $R_{13}$ und $R_{14}$ zusammen jeweils eine Methylendioxygruppe oder eine —CH=CH—CH=CH-Gruppe bilden.

Als neue Verbindungen der Formel (I) seien beispielhaft genannt:

4-β-Phenylethylaminomethyl-chroman,
4-β-Phenylethylaminomethyl-2,2-dimethylchroman,
4-β-Phenylethylaminomethyl-2-isopropylchroman,
4-β-Phenylethylaminoethyl-2-methyl-2-propyl-chroman,
4-β-Phenylethylaminomethyl-2,2-diethylchroman,
4-β-Phenylethylaminomethyl-2-spirocyclopentachroman,
4-β-Phenylethylaminomethyl-2-spirocyclohexachroman,
4-β-Phenylethylaminomethyl-2-hexylchroman,
4-β-Phenylethylaminomethyl-2-cyclopentylchroman,
4-β-Phenylethylaminomethyl-2-cyclohexylchroman,
4-β-Phenylethylaminomethyl-2-phenylchroman,
4-β-Phenylethylaminomethyl-2-benzylchroman,
4-β-Phenylethylaminomethyl-2H-chroman,
4-β-Phenylethylaminomethyl-2,2-dimethylchromen,
4-β-Phenylethylaminomethyl-2-spirocyclopentachromen,
4-β-Phenylethylaminomethyl-6-methyl-2-spirocyclopentachroman,
4-β-Phenylethylaminomethyl-7-methyl-2,2-dimethylchroman,
4-β-Phenylethylaminomethyl-7-methyl-2-spirocyclopentachroman,
4-β-Phenylethylaminomethyl-6-chlor-chroman,
4-β-Phenylethylaminomethyl-6-chlor-2-spirocyclopentachroman,
4-β-Phenylethylaminomethyl-6-methoxy-2,2-dimethylchroman,
4-β-Phenylethylaminomethyl-6-methoxy-2-spirocyclopentachroman,
4-β-Phenylethylaminomethyl-7-methoxy-2-spirocyclopentachroman,
4-β-Phenylethylaminomethyl-7-phenyl-2-spirocyclopentachroman,
4-β-Phenylethylaminomethyl-6,8-dichlor-2-spirocyclohexachroman,
4-β-Phenylethylaminomethyl-5-hydroxy-2,2-dimethylchroman,
4-β-Phenylethylaminomethyl-5-hydroxy-2-spirocyclopentachroman,
4-β-Phenylethylaminomethyl-6-hydroxy-2-spirocyclopentachroman,
4-β-Phenylethylaminomethyl-6-methyl-2-spirocyclohexachroman,
4-β-Phenylethylaminomethyl-7-methyl-2,2-dimethylchromen,
4-β-Phenylethylaminomethyl-6-chlor-2,2-dimethylchromen,
4-β-Phenylethylaminomethyl-6-chlor-2-spirocyclopnetachromen,
4-β-Phenylethylaminomethyl-6-methoxy-2H-chromen,
4-β-Phenylethylaminomethyl-6-methoxy-2-spirocyclopentachromen,
4-β-Phenylethylaminomethyl-7-methoxy-2,2-dimethylchromen,
4-β-Phenylethylaminomethyl-7-methoxy-2-isopropyl-2H-chromen,
4-β-Phenylethylaminomethyl-7-phenyl-2-spirocyclopentachromen,
4-β-Phenylethylaminomethyl-6,8-dichlor-2H-chromen,
4-β-Phenylethylaminomethyl-5-hydroxy-2-spirocyclopentachromen,
4-β-(4-Chlorpehnyl)-ethylaminomethyl-2-spirocyclopentachroman,
4-β-(3,4-Dichlorphenyl)-ethylaminomethyl-2,2-dimethylchroman,
4-β-(3-Methylphenyl)-ethylaminomethyl-2-isopropylchroman,
4-β-(4-Isopropylphenyl)-ethylaminomethyl-2,2-diethylchroman,
4-β-(3-Methoxyphenyl)-ethylaminomethyl-2-spirocyclopentachroman,
4-β-(4-Ethoxyphenyl)-ethylaminomethyl-2-spirocyclohexachroman,
4-β-(3,4-Dimethoxyphenyl)-ethylaminomethyl-chroman,
4-β-(3,4-Methylendioxyphenyl)-ethylaminomethyl-2,2,-dimethylchroman,
4-β-(3,4,5-Trimethoxyphenyl)-ethylaminomethyl-2-methyl-2-isopropyl-chroman,
4-β-(4-Hydroxypehnyl)-ethylaminomethyl-2,2-diethylchroman,
4-β-(4-Trifluormethylphenyl)-ethylaminomethyl-2-spirocyclopentachroman,

4-β-(3-Chlor-4-trifluormethylphenyl)-ethylaminomethyl-2-spirocyclohexachroman,
4-β-(1-naphthly)-ethylaminomethyl-2-spirocyclohexachroman,
4-β-(2-Naphthyl)-ethylaminomethyl-2,2-dimethylchroman,
4-β-(4-Chlorphenyl)-ethylaminomethyl-2-spirocyclohexachromen,
4-(β-Phenyl-α-methylethyl)aminomethyl-2-spirocyclohexachroman,
4-(β-Phenyl-α,α-dimethyl-ethyl)-aminomethyl-2,2-di-methylchroman,
4-(β-Phenyl-α,α-ethyl-ethyl)-aminomethyl-2-cyclopentylchroman,
4-(β-Phenyl-β-methyl-ethyl)-aminomethyl-2,2-diethylchroman,
4-(β-Phenyl-α-methylethyl)aminomethyl-2-spirocyclopentachromen,
4-δ-Phenylpropylaminomethyl-2-spirocyclopentachroman,
4-δ-Phenylpropylaminomethyl-2,2-dimethylchromen,
4-δ-Phenylpropylaminomethyl-6-methyl-2,2-dimethylchroman,
4-δ-(3,4-Methylendioxypehnyl)-propylaminomethyl-2-spirocyclopentachroman,
4-[δ-(4-Chlorphenyl)-α-methyl-propyl]-aminomethyl-2-isopropyl-2H-chromen,
4-β-Phenoxyethylaminomethyl-2,2-dimethylchroman,
4-β-Phenoxyethylaminomethyl-2-spirocyclopentachroman,
4-β-Phenoxyethylaminomethyl-2-spirocyclohexachroman,
4-β-Phenoxyethylaminomethyl-2-spirocyclopentachromen,
4-β-Phenoxyethylaminomethyl-6-methyl-2,2-dimethylchroman,
4-β-Phenoxyethylaminomethyl-7-methyl-2-spirocyclopentachroman,
4-β-Phenoxyethylaminomethyl-6-chlor-2-spirocyclohexachroman,
4-β-Phenoxyethylaminomethyl-6-methoxy-chroman,
4-β-Phenoxyethylaminomethyl-6-methoxy-2-phenylchroman,
4-β-Phenoxyethylaminomethyl-7-methoxy-2-isopropylchroman,
4-β-Phenoxyethylaminomethyl-7-phenyl-2,2-dimethylchroman,
4-β-Phenoxyethylaminomethyl-6,8-dichlor-2-spirocyclohexachroman,
4-β-Phenoxyethylaminomethyl-6,8-dimethyl-2-hexylchroman,
4-β-Phenoxyethylaminomethyl-5-hydroxy-chroman,
4-β-Phenoxyethylaminomethyl-6-hydroxy-2-spirocyclopentachroman,
4-β-Phenoxyethylaminomethyl-6-methyl-2,2-dimethylchromen,
4-β-(4-Chlorphenoxy)-ethylaminomethyl-chroman,
4-β-(3,4-Dichlorphenoxy)-ethylaminomethyl-2,2-dimethylchroman,
4-β-(3-Methylphenoxy)-ethylaminomethyl-2-methyl-2-isopropyl-chroman,
4-β-(4-Isopropylphenoxy)-ethylaminomethyl-2,2-diethylchroman,
4-β-(4-Ethoxyphenoxy)-ethylaminomethyl-2-spirocyclohexachroman,
4-β-(3,4-Dimethoxyphenoxy)-ethylaminomethyl-2-spirocyclopentachroman,
4-β-(3,4-Methylendioxyphenoxy)-ethylaminomethyl-2,2-dimethylchroman,
4-β-(3,4,5-Trimethoxyphenoxy)-ethylaminomethyl-2-cyclopentylchroman,
4-β-(4-Hydroxyphenoxy)-ethylaminomethyl-2,2-diethylchroman,
4-β-(4-Chlorphenoxy)-ethylaminomethyl-2H-chromen,
4-β-Phenoxy-α-methylethy)-aminomethyl-2-spirocyclohexachroman,
4-(β-Phenoxy-β-methyl-ethyl)-aminomethyl-2,2-diethylchroman,
4-(β-Phenoxy-α-methylethyl)-aminomethyl-2-spirocyclopentachromen,
4-(δ-Phenyl-α-methyl-propyl)-aminomethyl-2H-chromen,
4-(δ-Phenyl-α-methyl-propyl)-aminomethyl-2-spirocyclopentachromen,
4-(δ-Phenyl-α-methyl-propyl)-aminomethyl-2,2-dimethylchroman,
4-(δ-Phenyl-α-methyl-propyl)-aminomethyl-2-spirocyclopentachroman,
4-(δ-Phenyl-α-methyl-propyl)-aminomethyl-6-methyl-2,2-dimethylchroman,
4-(δ-Phenyl-α-methyl-propyl)-aminomethyl-6-chlor-2-spirocyclohexachroman,
4-(δ-Phenyl-α-methyl-propyl)-aminomethyl-7-methoxy-2-benzylchroman,
4-(δ-Phenyl-α-methyl-propyl)-aminomethyl-7-phenyl-2-cyclopentylchroman,
4-(δ-Phenyl-α-methyl-propyl)-aminomethyl-6,8-dichlorchroman,
4-(δ-Phenyl-α-methyl-propyl)-aminomethyl-6,8-dimethyl-2-cyclohexylchroman,
4-(δ-Phenyl-α-methyl-propyl)-aminomethyl-5-hydroxy-2-spirocyclopentachroman,
4-(δ-Phenyl-α-methyl-propyl)-aminomethyl-6-hydroxychroman,
4-(δ-3,4-Methylendioxyphenyl-α-methyl-propyl)-aminomethylchroman,
4-(δ-3,4-Methylendioxyphenyl-α-methyl-propyl)-aminomethyl-2,2-dimethylchroman,
4-(δ-3,4-Methylendioxyphenyl-α-methyl-propyl)-aminomethyl-2-isopropylchroman,
4-(δ-3,4-Methylendioxyphenyl-α-methyl-propyl)-aminomethyl-2,2-diethylchroman,
4-(δ-3,4-Methylendioxyphenyl-α-methyl-propyl)-aminomethyl-2-sprocyclopentachroman,
4-(δ-3,4-Methylendioxyphenyl-α-methyl-propyl)-aminomethyl-2-spirocyclohexachroman,
4-(δ-3,4-Methylendioxyphenyl-α-methyl-propyl)-aminomethyl-7-methyl-2-spirocyclopentachroman,
4-(δ-3,4-Methylendioxyphenyl-α-methyl-propyl)-aminomethyl-6-chlor-2-spirocyclopentachroman,
4-(δ-3,4-Methylendioxyphenyl-α-methyl-propyl)-aminomethyl-6-methoxy-2-spirocyclopentachroman,

4-(δ-3,4-Methylendioxyphenyl-α-methyl-propyl)-aminomethyl-7-methoxy-2-spirocyclopentachroman,
4-(δ-3,4-Methylendioxyphenyl-α-methyl-propyl)-aminomethyl-7-phenyl-2-spirocyclopentachroman,
4-(δ-3,4-Methylendioxyphenyl-α-methyl-propyl)-aminomethyl-2,2-dimethylchromen,
4-(δ-3,4-Methylendioxyphenyl-α-methyl-propyl)-aminomethyl-2-spirocyclopentachromen,
4-(δ-3-Trifluormethylphenyl-α-methyl-propyl)-aminomethyl-2-spirocyclopentachroman,
4-(δ-3,4-Dimethoxyphenyl-α-methyl-propyl)-aminomethyl-2-spirocyclopentachroman,
4-(δ-2,4-Dimethoxyphenyl-α-methyl-propyl)-aminomethyl-2-spirocyclopentachroman,
4-(δ-4-Chlorphenyl-α-methyl-propyl)-aminomethyl-2-spirocyclopentachroman,
4-(δ-3,4,5-Trimethoxypheny-α-methyl-propyl)-aminomethyl-2-spirocyclopentachroman,
4-(δ-3,4-Dimethoxyphenyl-α-methyl-propyl)-aminomethyl-2-spirocyclopentachromen,
4-(δ-4-Chlorpheny-α-methyl-propyl)-aminomethyl-2-spirocyclopentachromen,
4-β-Phenylamino-ethylaminomethyl-2,2-dimethylchroman,
4-β-Phenylamino-ethylaminomethyl-2-spirocyclopentachroman,
4-β-Phenylamino-ethylaminomethyl-2-spirocyclopentachromen,
4-β-(4-Chlorphenyl)-amino-ethylaminomethyl-2-spirocyclohexachroman,
4-β-(3,4-Dichlorphenyl)-amino-ethylaminomethyl-2,2-dimethylchroman,
4-β-(4-Isopropylphenyl)-amino-ethylaminomethyl-2-cyclohexylchroman,
4-β-(4-Ethoxyphenyl)-amino-ethylaminomethyl-2-spirocyclohexachroman,
4-(N-Phenylpiperazin-N'-yl-methyl)-2,2-dimethylchroman,
4-(N-Phenylpiperazin-N'-yl-methyl)-2-spirocyclopentachroman,
4-(N-Phenylpiperazin-N'-yl-methyl)-2-spirocyclohexachroman,
4-(N-Phenylpiperazin-N'-yl-methyl)-7-methyl-2,2-dimethylchroman,
4-(N-Phenylpiperazin-N'-yl-methyl)-6-chlor-2-spirocyclopentachroman,
4-(N-Phenylpiperazin-N'-yl-methyl)-6-methoxy-2-spirocycohexachroman,
4-(N-Phenylpiperazin-N'-yl-methyl)-7-methoxy-2,2-diethylchroman,
4-N-(4-Chlorphenyl)-piperazin-N'-yl-methyl-2-spirocyclohexachroman,
4-N-(3,4-Dichlorphenyl)-piperazin-N'-yl-methyl-2,2-dimethylchroman,
4-N-(3,4-Dichlorphenyl)-piperazin-N'-yl-methyl-2-hexylchroman,
4-N-(3,4-Dimethoxyphenyl)-piperazin-N'-yl-methyl-2-spirocyclopentachroman,
4-N-(3,4-Methylendioxyphenyl)-piperazin-N'-yl-methyl-2,2-dimethylchroman,
4-N-(3,4,5-Trimethoxyphenyl)-piperazin-N'-yl-methyl-2-methyl-2-propylchroman,
4-(N-Phenylpiperazin-N'-yl-methyl)-2H-chromen,
4-(N-Phenylpiperazin-N'-yl-methyl)-2,2-dimethylchromen,
4-(N-Phenylpiperazin-N'-yl-methyl)-6-methyl-2,2-dimethylchromen,
4-(N-Phenylpiperazin-N'-yl-methyl)-6-chlor-2-spirocyclopentachromen,
4-(N-Phenylpiperazin-N'-yl-methyl)-6-methoxy-2-spirocyclohexachromen,
4-(N-Phenylpiperazin-N'-yl-methyl)-7-methoxy-2,2-diethylchromen,
4-(N-Phenylpiperazin-N'-yl-methyl)-7-phenyl-2-benzyl-2H-chromen,
4-(N-Phenylpiperazin-N'-yl-methyl)-5-hydroxy-2,2-dimethylchromen,
4-N-(4-Chlorphenyl)-piperazin-N'-yl-methyl-2-spirocyclopentachromen,
4-N-(3,4-Dichlorphenyl)-piperazin-N'-yl-methyl-2,2-dimethylchromen,
4-N-(3-Methylphenyl)-piperazin-N'-yl-methyl-2-methyl-2-propylchromen,
4-N-(4-Isopropylphenyl)-piperazin-N'-yl-methyl-2,2-diethylchromen,
4-N-(3-Methoxyphenyl)-piperazin-N'-yl-methyl-2-spirocyclopentachromen,
4-N-(4-Ethoxyphenyl)-piperazin-N'-yl-methyl-2-spirocyclohexachromen,
4-N-(3,4-Dimethoxyphenyl)-piperazin-N'-yl-methyl-2H-chromen,
4-N-(3,4-Methylendioxyphenyl)-piperazin-N'-yl-methyl-2,2-dimethylchromen,
4-N-(3,4,5-Trimethoxyphenyl)-piperazin-N'-yl-methyl-2-isopropyl-2H-chromen,
4-N-(4-hydroxyphenyl)-piperazin-N'-yl-methyl-2,2-diethylchromen,
4-N-(Trifluormethylpehnyl)-piperazin-N-yl-methyl-2-spirocyclopentachromen,
4-N-(3-Chlor-4-trifluormethyl)-piperazin-N'-yl-methyl-2-spirocyclohexachromen.

Insbesondere seien folgende Verbindungen genannt:
4-[δ(3,4-Methylendioxyphenyl)-α-methyl]-aminomethyl-2-spirocyclopentachroman,
4-(β-Phenylethyl)-aminomethyl-2-spirocyclopentachroman,
4-N-3,4-Dichlorphenyl-piperazin-N'-yl-methyl-2,2-dimethyl-chromen,
4-β-Phenoxyethyl-aminomethyl-2-spirocyclopentachroman.

Die Erfindung betrifft weiterhin verschiedene Verfahren zur Herstellung der Verbindung der Formel (I).

Man setzt entweder

A) Chroman-4-carbaldehyde der Formel (II)

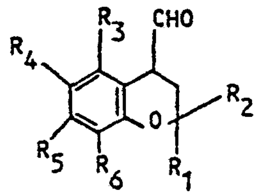

(II)

in welcher

$R_1$, $R_2$, $R_3$, $R_4$, $_5$ und $R_6$ die oben angegebene Bedeutung haben,

mit Aminen der Formel (III)

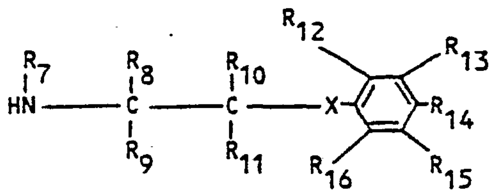

(III)

in welcher

$R_7$, $R_8$, $R_9$, $R_{10}$, $R_{11}$, $R_{12}$, $R_{13}$, $R_{14}$, $R_{15}$, $R_{16}$, $R_{17}$ und X die oben angegebene Bedeutung haben,

in Gegenwart von reduzierenden Angentien um, oder,

B) Amine der Formel (IV)

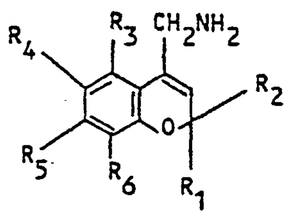

(IV)

oder der Formel (V)

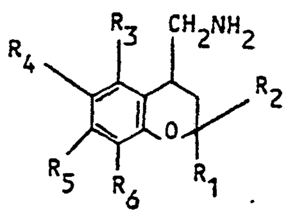

(V)

in welchen

$R_1$, $R_2$, $R_3$, $R_4$, $R_5$ und $R_6$ die oben angegebene Bedeutung haben,

mit Carbonylverbindungen der Formel (VI)

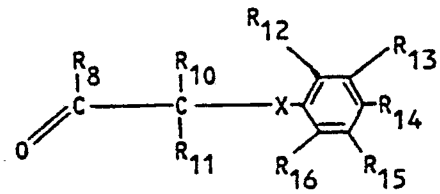

(VI)

in welcher

$R_8$ bis $R_{16}$ die oben angegebene Bedeutung haben, mit der Maßgabe, daß X nicht für $NR_{17}$ steht,

in Gegenwart von reduzierenden Angentien um, oder

C) Halogenide der Formel (VII)

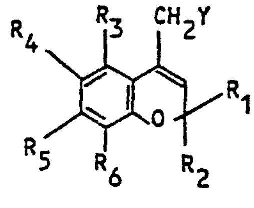

(VII)

in welcher

$R_1$, $R_2$, $R_3$, $R_4$, $R_5$ und $R_6$ die oben angegebene Bedeutung haben, und

Y für Brom oder Chlor steht, mit den Aminen der Formel (III) in Gegenwart eines säurebindenden Mittels um.

Als reduzierende Agentien, wie sie in den Verfahrensvarianten A und B eingesetzt werden können, seien beispielhaft komplexe Metallhydride gennant. Bevorzugt sind Alkaliborhydride, Alkalicyanoborhydride und/oder Alkalialanate, insbesondere Natrium- oder Lithiumverbindungen. Namentlich genannt seien Natriumborhydrid, Natriumcyanoborhydrid oder Lithiumalanat. Ebenso kann auch katalytisch erregter Wasserstoff bei erhöhten Drucken und Temperaturen eingesetzt werden.

Die reduzierenden Agentien können in äquivalenten Mengen bis zum Überschuß von 100%, bevorzugt von äquivalenten Mengen bis zum Überschuß von 20%, bezogen auf die eingesetzte Carbonylverbindung, eingesetzt werden.

Die in der Herstellungsvariante C eingesetzten säurebindenden Mittel sind bekannte Basen. Beispielhaft seien genannt: Erdalkali- oder Alkalihydroxide wie Natrium- und/oder Kaliumhydroxid, Erdalkali- oder Alkali-carbonate wie Natriumbicarbonat oder Kaliumcarbonat, organische Stickstoffbasen wie Triethylamin, Tributylamin oder Benzyltrimethylammoniumhydroxid.

Diese säurebindenden Mittel können in äquivalenten Mengen bis zum Überschuß von 100%, bevorzugt in äquivalenten Mengen bis zum überschuß von 20%, bezogen auf die eingesetzte Halogenverbindung, eingesetzt werden.

Die erfindungsgemäßen Reaktionen werden in Lösungsmitteln durchgeführt. Als Lösungsmittel kommen alle für die jeweilige Reaktion inerten Lösungsmittel in Frage, vorzugsweise seien genannt:

Alkohole wie Methanol, Ethanol, Isopropanol oder tert.- Butanol, Ether wie Diethylether, Diisopropylether, Tetrahydrofuran oder Dioxan, Kohlenwasserstoffe wie Hexan, Cyclohexan, Benzol oder Toluol, Chlorkohlenwasserstoffe wie Methylenchlorid, Chloroform, Tetrachlorkolenstoff oder Chlorbenzol oder Mischungen solcher Lösungsmittel.

Die Reaktionstemperaturen können in einem größeren Bereich variiert werden. Im allgemeinen arbeitet man zwischen −50°C und 150°C, vorzugsweise von −10 bis 120°C.

Bei der Durchführung der erfindungsgemäßen Verfahren setzt man bevorzugt bezogen auf 1 Mol der Chroman- oder Chromenverbindung 0,5 bis 2 Mole des Amins der Formel (III) oder der Carbonylverbindung der Formel (VI) um. Besonders bevorzugt beträgt das Molverhältnis der Reaktionspartner 1:1. Benutzt man einen Überschuß, so setzt man bevorzugt einen Überschuß an Amin der Formel (III) oder an Carbonylverbindung der Formel (VI) ein.

Die Reaktionsprodukte können durch Destillation, Auskristallisieren, Einengen und Umlösen oder chromatographische Trennung gewonnen werden.

Die Herstellung der Chroman-4-aldehyde der Formel (II) erfolgt durch Hydroformylierung aus 2H-Chromenen gemäß folgender Gleichung:

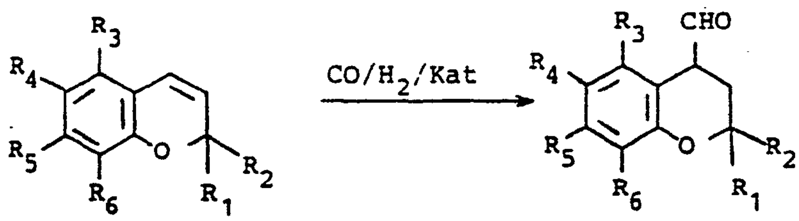


Das Verfahren ist dadurch gekennzeichnet, daß die 2H-Chromene mit Kohlenmonoxid und Wasserstoff in Gegenwart von Metallkatalysatoren aus der 8. Nebengruppe des Periodensystems bei Temperaturen von 80 bis 250°C und Drucken von 20—1000 bar umgesetzt werden.

Als Beispiele für die Chroman-4-carbaldehyde seien genannt:

4-Formylchroman, 4-Formyl-2-methylchroman, 4-Formyl-2,2-dimethylchroman, 4-Formyl-2-propylchroman, 4-Formyl-2-isopropylchroman, 4-Formyl-2,2-diethylchroman, 4-Formyl-2-methyl-2-propylchroman, 4-Formyl-2-hexylchroman, 4-Formyl-2-cyclopentylchroman, 4-Formyl-2-cyclohexylchroman, 4-Formyl-2-spirocyclopentachroman, 4-Formyl-2-spirocyclohexachroman, 4-Formyl-6-methyl-2-spirocyclopentachroman, 4-Formyl-7-methyl-2-spirocyclopentachroman, 4-Formyl-6,8-dimethyl-2-spirocyclopentachroman, 4-Formyl-6-chlor-2-spirocyclopentachroman, 4-Formyl-6-methoxy-2-spirocyclopentachroman, 4-Formyl-7-methoxy-2-spirocyclopentachroman, 4-Formyl-7-isopropoxy-2-spirocyclopentachroman, 4-Formyl-7-pehnoxy-2-spirocyclopentachroman, 4-Formyl-7-benzyloxy-2-spirocyclopentachroman, 4-Formyl-phenyl-2-spirocyclopentachroman, 4-Formyl-6-methyl-2,2-dimethylchroman, 4-Formyl-6-chlor-2,2-dimethylchroman, 4-Formyl-7-methoxy-2,2-dimethylchroman, 4-Formyl-6-methyl-2-spirocyclohexachroman, 4-Formyl-7-methoxy-2-spirocyclohexachroman.

Die bei der Herstellung der erfindungsgemäßen Verbindung eingesetzten Amine der Formel (III) sind zum großen Teil bekannt (vgl. z.B. Beilsteins Handbuch der organischen Chemie *6*, 172, III 639, IV 663; *12*;, 543, 1096, 1145, I 473, 494, II 287, 591, 623, III/IV 49), sie können im übrigen nach analogen Verfahren erhalten werden.

Als Beispeiele für die Amine der Formel (III) seien genannt:

2-Phenylethylamin, 2-(2-Chlorphenyl)-ethylamin, 2-(4-Chlorphenyl)-ethylamin, 2-(3,4-Dichlorphenyl)-ethylamin, 2-(3-Methylphenyl)-ethylamin, 2-(2,4-Dimethylphenyl)-ethylamin, 2-(2-Methyl-4-chlorphenyl)-ethylamin, 2-(4-Hydroxyphenyl)-ethylamin, 2-(4-Methoxy-phenyl)-ethylamin, 2-(3,4-Dimethoxyphenyl)-ethylamin, 2-( -Naphthyl)-ethylamin, 2-(β-Naphthyl)-ethylamin, 2-(3,4-Dioxymethylenphenyl)-ethylamin, 3-Phenyl-propylamin, 2-Phenylpropyl-amin, 3-(4-Chorphenyl)-propylamin, 2-(4-Chlorphenyl)-propylamin, 3-(4-Methylphenyl)-propylamin, 2-(3-Methylphenyl)propylamin, 3-(4-Methoxyphenyl)-propylamin, 2-(3,4-Dimethoxyphenyl)-propylamin, 3-(3,4-Methylendioxyphenyl)propylamin, 2-Amino-4-phenyl-butan, 2-Amino-4-(4-chlorphenyl)-butan, 2-Amino-4-(4-methylphenyl)-butan, 2-Amino-4-(4-trifluormethylphenyl)-butan, 2-Amino-4-(4-isopropylphenyl)-butan, 2-Amino-4-(3,4-dichlorphenyl)-bvutan, 2-Amino-4-(2-methoxyphenyl)-butan, 2-Amino-4-(3-methoxyphenyl)-butan, 2-Amino-4-(4-methoxyphenyl)-butan, 2-Amino-4-(4-propoxyphenyl)-butan, 2-Amino-4-(3,4-dimethoxyphenyl)-butan, 2-Amino-4-(3,4-methylendioxyphenyl)-butan, 3-Amino-5-phenyl-pentan, 3-Amino-5-(3,4-methylendioxyphenyl)-pentan, 2-Phenyl-1-methyl-ethyl-amin, 2-Phenyl-1,1-dimethyl-ehtylamin, 2-Phenyl-1,1-diethyl-ethylamin, 2-Phenyl-2,2-dimethyl-enthylamin, 2-Phenyl-2,2-diethyl-ethylamin, 2-Phenyl-N-methyl-ethyl-amin, 2-Phenyl-N-ethyl-ethylamin, 2-Phenyl-N-isopropylethylamin, 2-(4-Chlorphenyl)-N-methyl-ethylamin, 2-(4-Methoxyphenyl)-N-methyl-ethylamin, 2-(4-Isopropylphenyl)-N-methyl-ethylamin, 2-Phenoxyethylamin, 2-(4-Methylphenoxy)-ethylamin, 2-(4-tert.-Butylphenoxy)-ethylamin, 2-(4-Chlorphenoxy)-ethylamin, 2-(4-Trifluormethylphenoxy)-ethylamin, 2-(3-Ethoxyphenoxy)-ethylamin, 2-(4-Methoxyphenoxy)-ethylamin, 2-(4-isopropoxyphenoxy)-ethylamin, 2-Phenoxy-propylamin, 2-(4-Fluorphenoxy)-propylamin, 2-Phenoxybutanamin, 2-Phenoxy-N-methyl-ethylamin, 2-Phenoxy-N-ethyl-ethylamin, 2-Phenoxy-N-isopropylethylamin, N-Phenyl-ethylendiamin, N-(4-chlorphenyl)-ethylendiamin, N-(3,4-Dichlorphenyl)-ethylendiamin, N-(4-Methylphenyl)-ethylendiamin, N-(4-Isopropylphenyl)-ethylendiamin, N-(4-Trifluormethylphenyl)-ethylendiamin, N-(4-Hydrophenyl)-ethylendiamin, N-(2-Methoxyphenyl)-ethylendiamin, N-(4-Ethoxyphenyl)-ethylendiamin, N-Phenyl-N'-methyl-ethylendiamin, N-Phenyl-N'-ethyl-ethylendiamin, N-(4-Chlorphenyl)-N'-isopropyl-ethylendiamin, N-Phenyl-N-methyl-ethylendiamin, N-Phenyl-N-methyl-N'-ethylendiamin, 2-Amino-3-anilino-propane, 3-Amino-4-anilino-butan, 2-Amino-2-methyl-3-anilino-propan, 2-Amino-2-methyl-3-(4-chloranilino)-propan, 1-Amino-2-anilono-propan, 1-Amino-2-anilino-butan, 1-Amino-2-methyl-2-anilino-propan, 1-Amino-2-methyl-2-(4-methoxyanilino)-propan, N-Phenylpiperazin, N-(2-Chlorphenyl)-piperazin, N-(4-Chlorphenyl)-piperazin, N-(3,4-Dichlorphenyl)-p-iperazin, N-(4-trifluormethylphenyl)-piperazin, N-(3-Trifluormethylphenyl)-piperazin, N-(3-Trifluormethyl-4-chlorphenyl)-piperazin, N-(2-Methoxyphenyl)-piperazin, N-(2-Ethoxyphenyl)-piperazin, N-(2-Isopropoxyphenyl)-piperazin, N-(3-methoxyphenyl)-piperazin, N-(4-Methoxyphenyl)-piperazin, N-(3,4-Dimethoxyphenyl)-piperazin, N-(3,4-Methylendioxyphenyl)-piperazin, N-(4-Methylphenyl)-piperazin, N-(3,4-Dimethylphenyl)-piperazin, N-(2,4-Dimethylphenyl)-piperazin, N-(2,5-Dimethylphenyl)-piperazin, N-(4-Ethylphenyl)-piperazin, N-(4-Isopropylphenyl)-piperazin, N-(4-tert.-Butylphenyl)-piperazin, N-(4-Cyclohexylphenyl)-piperazin.

Die bei der Herstellung der erfindungsgemäßen Verbindungen eingesetzten Amine der Formel (IV) sind teils bekannt (J. med. Chem. *1982*, 393). Sie können im übrigen durch analoge Verfahrensweisen dargestellt werden.

Man geht dabei von Chroman-4-onen aus, die mit Trimethylsilylcyanid, wie im folgenden Schema angegeben, umgesetzt werden:

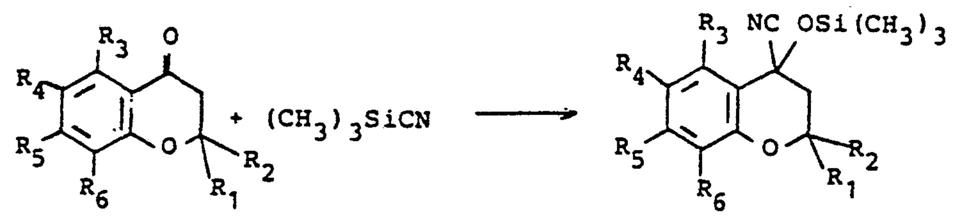


Die hierbei entstehenden 4-Cyan-4-trimethylsilyloxy-chromane können mit Lithiumaluminiumhydrid zu 4-Amino-methyl-4-hydroxychromanen hydriert werden, wie in folgendem Schema angegeben:

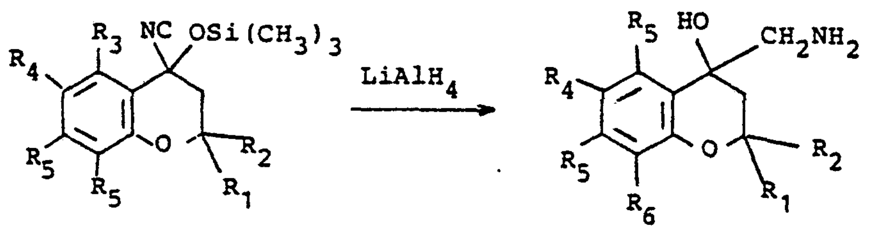

Die 4-Aminomethyl-4-hydroxy-chromane können durch wasserabspaltende Mittel in die 4-Aminomethyl-2H-chromene der Formel (IV), wie im folgenden Schema gezeigt, übergeführt werden.

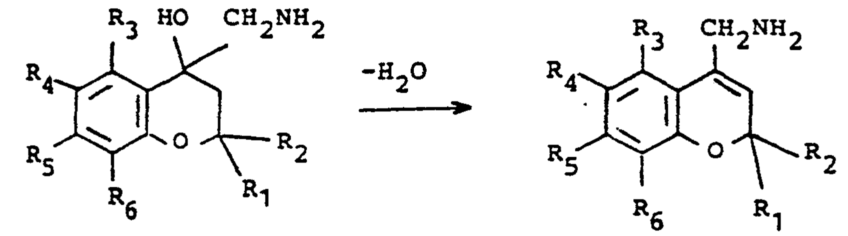


Aus den Aminomethylchromenen lassen sich die Amino-methylchromane durch Hydrierung wie in folgendem Schema gewinnen:

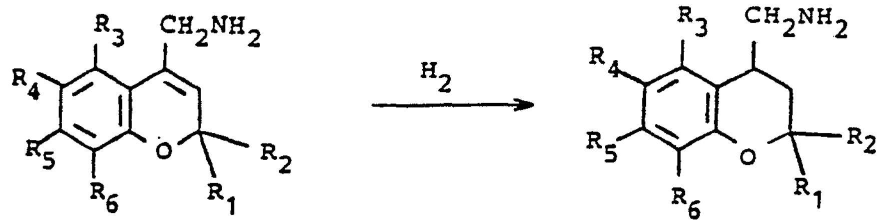


Man kann aber auch aus den 4-Cyan-4-trimethylsilyloxychromanen mit Hilfe von Phosphoroxychlorid Trimethylsilanol abspalten und so 4-Cyan-2H-chromene gewinnen, wie im folgenden Schema angegeben (analog zu einer Verfahrensweise aus Chemistry Letters *1979*, 1427):

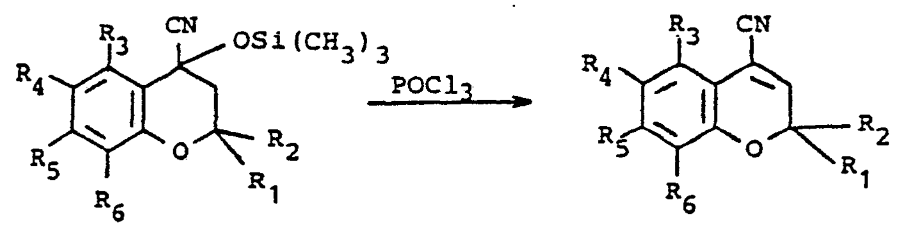


Die 4-Cyan-2H-chromene können dann über die 4-Cyanchromane direkt oder stufenweise zu den 4-Aminomethylchlormanen der Formel hydriert werden, wie im folgenden Schema dargestellt:

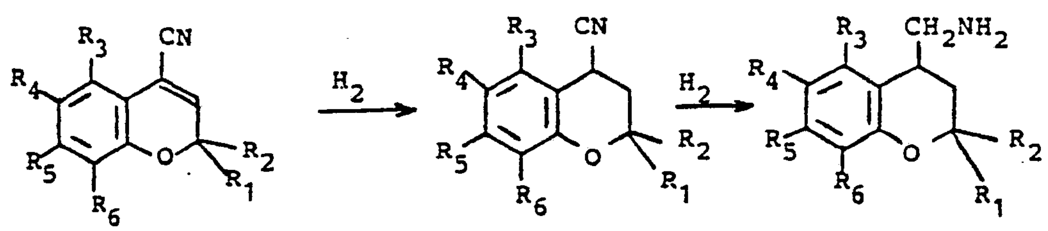


Als Beispiele für die 4-Aminomethylchromene-2H seien genannt:

4-Aminomethyl-2H-chromen, 4-Aminomethyl-2-methyl-2H-chromen, 4-Aminomethyl-2,2-dimethyl-chromen, 4-Aminomethyl-2-propyl-2H-chromen, 4-Aminomethyl-2-isoporopyl-2H-chromen, 4-Aminomethyl-2,2-diethyl-chromen, 4-Aminomethyl-2-methyl-2-propyl-chromen, 4-Aminomethyl-2-hexyl-2H-chromen, 4-Aminomethyl-2-cyclopentyl-2H-chromen, 4-Aminomethyl-2-cyclohexyl-2H-chromen, 4-Aminomethyl-2-spirocyclopentachromen, 4-Aminomethyl-2-spirocyclohexachromen, 4-Aminomethyl-6-methyl-2-spirocyclopentachromen, 4-Aminomethyl-7-methyl-2-spirocyclopentachromen, 4-Aminomethyl-6,8-dimethyl-2-spirocyclopentachromen, 4-Aminomethyl-6-chlor-2-spirocyclopentachromen, 4-Amino-methyl-6-methoxy-2-spirocyclopentachromen, 4-Aminomethyl-7-methoxy-2-spirocyclopentachromen, 4-Aminomethyl-7-isopropoxy-2-spirocyclopentachromen, 4-Aminomethyl-7-phenoxy-2-spirocyclopenta-chromen, 4-Aminomethyl-7-benzyloxy-2-spirocyclopentachromen, 4-Aminomethyl-7-phenyl-2-spirocyclo-pentachromen, 4-Aminomethyl-6-methyl-2-spirocyclopentachromen, 4-Aminomethyl-6-chlor-2-spirocyclo-hexachromen, 4-Aminomethyl-7-methoxy-2-spirocyclopentachromen, 4-Aminomethyl-6-methyl-2,2-dimethylchromen, 4-Aminomethyl-7-methoxy-2,2-dimethylchromen.

Die bei der Herstellung der erfindungsgemäßen Verbindung eingesetzten Amine der Formel (V) sind teils bekannt (J. med. Chem. *1982*, 393). Sie können im übrigen nach analogen Verfahrensweisen, beispielsweise wie oben beschrieben, gewonnen werden.

Als Beispiele für die 4-Aminomethylchromane seien genannt:

4-Aminomethyl-chroman,
4-Aminomethyl-2-methyl-chroman,
4-Aminomethyl-2,2-dimethyl-chroman,
4-Aminomethyl-2-propyl-chroman,
4-Aminomethyl-2-isopropyl-chroman,
4-Aminomethyl-2,2-diethyl-chroman,
4-Aminomethyl-2-methyl-2-propyl-chroman,
4-Aminomethyl-2-hexyl-chroman,
4-Aminomethyl-2-cyclopentyl-chroman,
4-Aminomethyl-2-cyclohexyl-chroman,
4-Aminomethyl-2-spirocyclopenta-chroman,
4-Aminomethyl-2-spirocyclohexa-chroman,
4-Aminomethyl-6-methyl-2-spirocyclopenta-chroman,
4-Aminomethyl-7-methyl-2-spirocyclopenta-chroman,
4-Aminomethyl-6,8-dimethyl-2-spirocyclopenta-chroman,
4-Aminomethyl-6-chlor-2-spirocyclopenta-chroman,
4-Aminomethyl-6-methoxy-2-spirocyclopenta-chroman,
4-Aminomethyl-7-methoxy-2-spirocyclopenta-chroman,
4-Aminomethyl-8-isopropoxy-2-spirocyclopenta-chroman,
4-Aminomethyl-7-phenoxy-2-spirocyclopenta-chroman,
4-Aminomethyl-7-benzyloxy-2-spirocyclopenta-chroman,
4-Aminomethyl-7-phenyl-2-spirocyclopenta-chroman,
4-Aminomethyl-6-methyl-2-spirocyclopenta-chroman,
4-Aminomethyl-6-chlor-2-spirocyclopenta-chroman,
4-Aminomethyl-7-methoxy-2-spirocyclopenta-chroman,
4-Aminomethyl-6-methyl-2,2-dimethylchroman,
4-Aminomethyl-7-methoxy-2,2-dimethylchroman.

Die bei der Herstellung des erfindungsgemäßen Verfahrens verwendeten Carbonylverbindungen der Formel (VI) sind zum großen Teil bekannt (vgl. z.B. Beilsteins Handbuch der organischen Chemie *6*, 151, II 152; 7, 292, 303, 304, 314, I 154, 161, 162, 167, II 226, 233, 236, 243). Sie können im übrigen nach analogen Verfahren hergestellt werden.

Als Beispiele für die Carbonylverbindungen seien genannt:

Phenylacetaldehyd, 2-Chlorphenylacetaldehyd, 3-Chlorphenylacetaldehyd, 4-Chlorphenylacetaldehyd, 3,4-Dichlorphenylacetaldehyd, 4-Methylphenylacetaldehyd, 4-Isopropylphenylacetaldehyd, 3-Methoxyphenylacetaldehyd, 4-Ethoxyphenylacetaldehyd, 3,4-Dimethxyphenylacetaldehyd, 3,4-Methylendioxyphenylacetaldehyd, α-Penylpropionaldehyd, β-Phenylpropionaldehyd, β-(4-Chlorphenyl)-propionaldehyd, β-(4-Trifluormethylphenyl)propionaldehyd, β-(4-trifluormethylphenyl)-propionaldehyd, β-(4-Methoxyphenyl)propionaldehyd, β-(3,4-Dimethoxyphenyl)-propionaldehyd, β-(3,4-Methylendioxyphenyl-propionaldehyd, Phenoxy-acetaldehyd, 4-Chlorphenylacetaldehyd, 3,4-Dichlor-phenoxyacetaldehyd, 2,4-Dichlorphenylacetaldehyd, 3,4-Dimethylphenoxyacetaldehyd, 4-Methoxyphenoxyacetaldehyd, 3,4-Dimethoxyphenoxyacetaldehyd, 3,4-Methylendioxyphenylacetaldehyd, Phenylaceton, 4-Chlorphenylaceton, 3,4-Dichlorphenylaceton, 4-Methylphenylaceton, 4-Methoxyphenylaceton, 3,4-Dimethoxyphenylaceton, 3,4-Methylendioxyphenylaceton, Benzylacton, (4-Chlorbenzyl)-aceton, (4-Trifluormethylbenzyl)-aceton, (4-Methoxybenzyl)-aceton, (3,4-Dimethoxybenzyl)-aceton, (3,4-Methylendioxybenzyl)-aceton, Phenoxyaceton, (4-Chlorphenoxy)-aceton, (3,4-Dichlorphenoxy)-aceton, (2,5-Dimethylphenyoxy)-aceton, (4-Methoxyphenoxy)-aceton, (3,4-Dimethoxyphenoxy)-aceton, (3,4-Methylendioxyphenyl)-aceton.

Die bei der Herstellung der erfindungsgemäßen Verbindungen eingesetzten Halogenide der Formel (VII) sind zum Teil bekannt (Tetrahedron *23*, 1893 (1967)). Sie können im übrigen auf analogem Weg oder aus 4-Methyl-chromenen-2H [Heterocyclic Compounds, Vol. 31. Ed. G.P. Ellis (New York 1977), p 11 ff.] durch Halogenierung mit N-Brom- oder N-Chlorsuccinimid gewonnen werden (Wohl-Ziegler-Reaktion).

Als Beispiele für die 4-Halogenmethylchromene seien gennant:

4-Chlormethyl-2H-chromen, 4-Brommethyl-2-methyl-2H-chromen, 4-Brommethyl-2,2-dimethylchromen, 4-Bromethyl-2-isopropyl-2H-chromen, 4-Chlormethyl-2,2-diethylchromen, 4-Chlormethyl-2-methyl-2-propyl-chromen, 4-Brommethyl-2-hexyl-2H-chromen, 4-Brommethyl-2-cyclopentyl-2H-chromen, 4-Brommethyl-2-cyclohexyl-2H-chromen, 4-Brommethyl-2-spirocyclopentachromen, 4-Brommethyl-2-spirocyclohexachromen, 4-Brommethyl-6-methyl-2-spirocyclopentachromen, 4-Brommethyl-7-methyl-2-spirocyclopentachromen, 4-Brommethyl-6,8-dimethyl-2-spirocyclopentachromen, 4-Brommethyl-3-chlor-2-spirocyclopentachromen, 4-Brommethyl-6-methoxy-2-spirocyclopentachromen, 4-Brommethyl-7-methoxy-2-spirocyclopentachromen, 4-Brommethyl-7-isopropoxy-2-spirocyclopentachromen, 4-Brommethyl-7-penoxy-2-spirocyclopentachromen, 4-Brommethyl-7-benzyloxy-2-spirocyclopentachromen, 4-Brommethyl-7-phenyl-2-spirocyclopentachromen, 4-Brommethyl-6-methyl-2,2-dimethylchromen, 4-Brommethyl-6-chlor-2,2-dimethylchromen, 4-Brommethyl-7-methoxy-2,2-dimethylchromen, 4-Brommethyl-6-methyl-2-spirocyclohexachromen, 4-Brommethyl-7-methoxy-2-spirocyclohexachromen.

Die erfindungsgemäßen Chroman- und Chromen-Derivate, zeigen überraschenderweise eine antihypertensive Wirkung und können deshalb in freier Form oder in Form ihrer pharmazeutisch unbedenklichen Säureadditions-salze als Arzneimittel eingesetzt werden.

Die neueun Verbindungen haben ein breites und vielseitiges pharmakologisches Wirkungsspektrum und überraschend lange Wirkungsdauer.

Im einzelnen konnten im Tierexperiment folgende Hauptwirkungen nachgewiesen werden:

1. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. dem Zentralnervensystem) mainfestieren. Die Verbindungen eignen sich daher besonders als Cerebraltherapeutika.

2. Die Verbindungen senken den Blutdruck von normotonen und hypertonen Tieren und können somit als antihypertensive Mittel verwendet werden.

Die erfindungsgemäßen Verbindungen eignen sich aufgrund dieser Eigenschaften zur Prophylaxe der akuten und chronischen ischämischen Herzkrankheit im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lössungsmittel. Hierbei soll die thereapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittle, wie Paraffine (z.B. Erdölfraktionen), pflanzliche Öle (z.B. Erdnuß-/Sesam-Öl), Alkohole (z.B. Ethylalkohol, Glycerin), Glykole (z.B. Propylenglykol, polyethylenglykol), feste Trägerstoffe, wie z.B. natürliche Gesteinsmehle (z.B. Kaoline, Tonerden, Talkum, Kriede), synthetische Gesteinsmehle, (z.B. hockdisperse Kieselersäure, Silikate), Zucker (z.B. Roh-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzungsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffgen, wie Stärke, verzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,5 bis 30 mg/kg, vorzugsweise 1 bis 10 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und der individuellem Verhalten gegenüber dem Medikament bzw. der Art von dessen Formulierung und dem Zeipunkt bzw. Intervall, zu welchem die Verabreichung erfolgt. So kann es in einigen Fällen ausreichend sein, mit weniger als der vorgenannten Mindestmenge auszukommen, während in anderen Fällen die gemannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungspielraum vergesehen. Sinngemäß gelten hierbei auch die obigen Ausführungen.

Herstellungsbeispiele für Zwischenprodukte der Herstellungsvariante B

1)

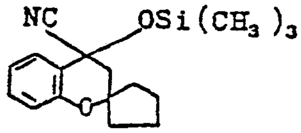

In eine Mischung von 528 g (4,8 m) Trimethylsilylcyanid und 969 g (4,8 m) 2-Spirocyclopentachromanon-4 wird unter Kühlung und Rühren 30 ml Bortrifluorid-Etherat so zugetropft, daß die Temperatur 40—50°C nicht übersteigt; anschließend 5 Stunden nachrühren bei Raumtemperatur und über Nacht stehen lassen. Dann wird im Hochvakuum entgast bei einer Temperatur von 50°C. Man erhält so das Produkt als viskoses öl in 85—95% GC-Ausbeute. Für alle weiteren Umsetzungen ausreichend rein.

Auf ähnliche Weise wurden hergestellt:

2)

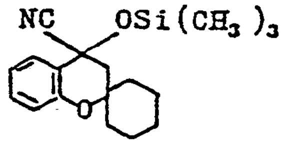

Schmp. 65—7°C

3)

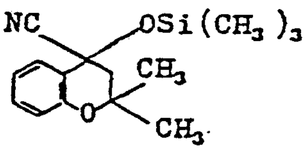

Schmp. 36—8°C

4)

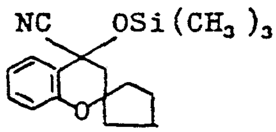

Schmp. 68—70°C

5)

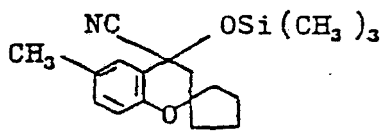

Schmp. 66—8°C

6)

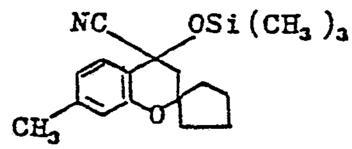

Schmp. 71—3°C

7)

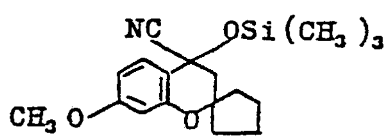

Oel

8)

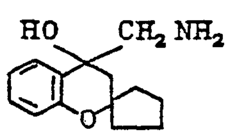

16,4 g Lithium-aluminium-hydrid werden mit 600 ml trockenem Tetrahydrofuran vorgelegt und eine Lösung von 120 g 2-Spirocyclopenta-4-cyan-4-trimethylsilyloxy-chroman (89 %ig) in 120 ml trockenem Tetrahydrofuran unter Rühren und Kühlung mit Aceton/Trockeneis zugetropft. (Stickstoffatmosphäre, Feuchtigkeitsausschaltung, Innen-temperatur <20°C).

Man läßt unter Rühren auf Raumtemperatur kommen, erhitzt dann 3 Stunden zum Rückfluß und läßt abkühlen. 16.4 g Wasser werden vorsichtig eingetropft, anschließend 49,2 g 15 %ige Kalilauge. Nach Stehen über Nacht saugt man ab, engt die Mutterlauge in Vakuum ein und reibt den Rückstand mit 135 ml

Ether ab. Das anfallende weiße kristalline Produkt saugt man ab. Man erhält 35—37 g. Die etherische Mutterlauge wird eingeengt und in 100 ml Methanol aufgenommen, mit wenig Kaliumhydroxyd 1 Stunde gekocht und aufgearbeitet. Auf diese Weise vervollständigt man die Silyletherhydrolyse. Man erhält so insgesamt 66 g weiße Kristalle (82% der Theorie); F 119°C.

Auf ähnliche Weise wurden hergestellt:

9) 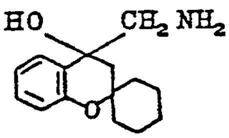 Schmp. 100°C

10) 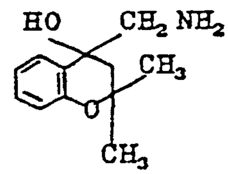 Schmp. 73°C

11) 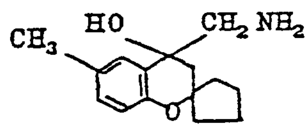 Schmp. 98–100°C

12) 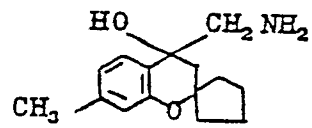 Schmp. 94°C

13) 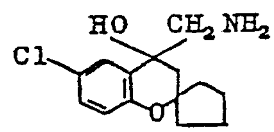 Schmp. 104–6°C

14) 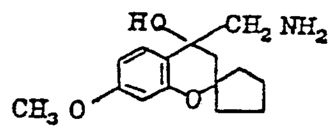 Schmp. 93°C

15) 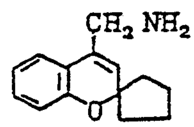

2 Ltr. Toluol und 190 g Toluolsulfonsäure-monohydrat werdem am Wasserabscheider bis zur Beendigung der azeotropen Trocknung gekocht. In die abgekühlte Mischung gibt man 233 g (1 m) 2-Spiro-cyclopenta-4-hydroxy-4-aminomethylchroman und erhitzt 5 h zum Sieden, wobei ca. 18 ccm Wasser abgeschieden werden. Nach Abkühlen wird von einem geringen Feststoffanteil abgesaugt und die Toluolphase mit überschüssiger konzentrierter Natronlauge verrührt. Nach dem Phasentrennen wird die Toluollösung mit Wasser gewaschen und wie üblich aufgearbeitet. 140 g (65% der Theorie), Kp. 147—170°C / (0,7—1,2 mm Hg) 93—160 Pa.

Auf ähnliche Weise wurden hergestellt:

16) 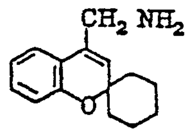 Kp. 132–8°C /(0,4 mm Hg) 53 Pa

17) 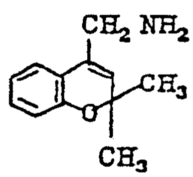 Kp. 120–5°C /(0,5 mmHg) 67 Pa

18) 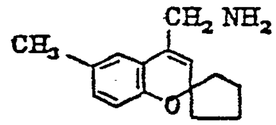 Kp. 139–144°C /(0,2 mmHg) 27 Pa

19) 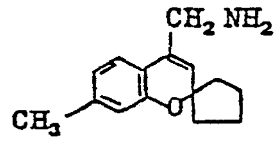 Kp. 145°C /(0,25 mmHg) 33 Pa

20) 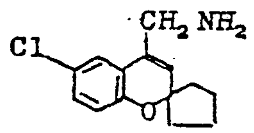 Kp. 147–9°C /(0,3 mmHg) 40 Pa

21) 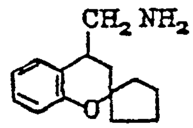

50 g 2-Spirocyclopenta-4-aminomethyl-chromen-3 werden zusammen mit 300 ml Methanol und 10 g Raney-Nickel bei 60°C und 100 bar $H_2$-Druck während ca. 4,5 h in einem Druckautoklaven katalytisch hydriert. Danach wird vom Katalysator abfiltriert, eingeengt und fraktioniert.

Kp. 150—54°C/(0,9 mmHg) 120 Pa; 40 g farblose Flüssigkeit (ca. 80% der Theorie)

Auf ähnliche Weise wurden hergestellt:

22) 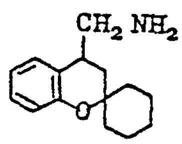 135–40°C /(0,5 mmHg) 67 Pa

23) 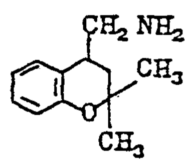 110–12°C /(0,3 mmHg) 40 Pa

24) 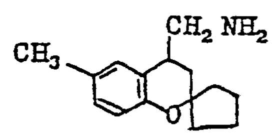 132–36°C /(0,3 mmHg) 40 Pa

25) 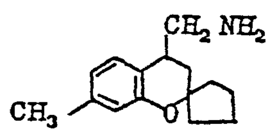 135–8°C /(0,3 mmHg) 40 Pa

26) 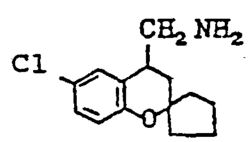 144–8°C /(0,3 mmHg) 40 Pa

27) 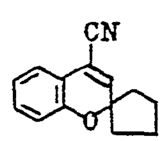

429 g 2-Spirocyclopenta-4-cyan-4-trimethylsilyloxychroman (89 %ig) werden in 2,2 Ltr. abs. Pyridin vorgelegt. Man tropft 694 g Phosphoroxychlorid zu, dabei schwach exotherme Reaktion. Es wird *langsam* zum Sieden erhitzt, da die Reaktion beim Erwärmen stärker in Gang kommt. Nach 10 Stunden Rückflußkochen und Abkühlen wird vorsichtig auf eine Mischung von ca. 6 Ltr. gestoßenem Eis und 1 Ltr. konz.

Salzsäure gegossen. Das anfallende Präzipitat wird abgesaugt und mit Wasser gewaschen, in Toluol aufgenommen, mit Aktivkohle durchgeschüttelt und mit Natriumbicarbonatlösung ausgeschüttelt. Nach Trocknen mit Natriumsulfat dampft man das Toluol im Vakuum ab und kristallisiert den Rückstand aus Methylcyclohexan unter Zusatz von Aktivkohle um, 180 g (67% der Theorie) als leicht graue Kristalle vom Schmelzpunkt 50°C. Das Produkt kann destilliert werden.

Kp 135°C/(0,2 mmHg) 27 Pa. Schmelzpunkt der rein weißen Kristalle 58—59°C.

28) 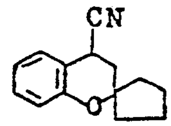

400 g 2-Spirocyclopenta-4-cyan-chromen-3 werden in 1800 ml Tetrahydrofuran über 40 g Palladiumkohle (5 %ig) bei 40—45°C und 50 bar druckhydriert. Nach der üblichen Aufarbeitung erhält man 360 g (~ 90% der Theorie) weiße Kristalle vom Kp. 131—5°C/(0,3 mmHg) 40 Pa und Schmp. 44—5°C.

29) 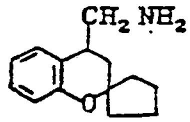

50 g 2-Spirocyclopenta-4-cyanchroman werden zusammen mit 200 ml Methanol, 50 ml flüssigem Ammoniak und 10 g Raney-Kobalt bei 90°C und 100 bar $H_2$-Druck in einem Druckautoklaven während ca. 3,5 h katalytisch hydriert. Danach wird vom Katalysator abfiltriert, eingeengt und fraktioniert.

Kp 130—140°C/(0,35 mmHg) 47 Pa 40 g farblose Flüssigkeit (ca. 80% der Theorie) (identisch mit dem Produkt aus Beispiel 21).

30) Herstellungsbeispiele für Zwischenprodkte der Herstellungsvariante C

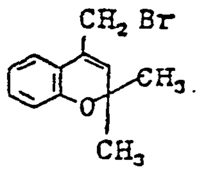

176 g (1 m) 2,2,4-Trimethylchromen werden in 1 Ltr. über Phosphorpentoxid getrocknetem Tetrachlorkohlenstoff gelöst und mit 178 g (1 m) N-Brom-succinimid sowie 2 g Azo-bis-isobutyronitril versetzt. Die Mischung wird langsam zum Sieden erhitzt und 1 Stunde gekocht. Nach dem Abkühlen wird vom Niederschlag abgesaugt (Succinimid). Die Mutterlauge wird im Vakuum eingeengt; 255 g eines braunen Öls, GC-Gehalt 89 %ig (~ 90% der Theorie), das für weitere Umsetzungen direkt eingesetzt werden kann. Das Produkt destilliert bei 105—9°C/(0,28 mmHg) 37 Pa. Es kann als Pyridiniumbromid (Schmp. 186—7°C) charakterisiert werden.

Auf ähnliche Weise hergestellt:

31) 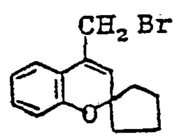

Oel, nicht destillationsstabil, Schmp. des Pyridinium-bromids 165—7°C

Erfindungsgemäße Beispiele

32) Herstellung nach Verfahrensvariante A

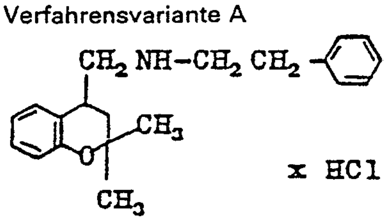

x HCl

19,0 g (0,1 m) 2,2-Dimethyl-4-formyl-chroman werden in 100 ml Methanol unter Rühren und schwacher Kühlung bei RT mit 12,1 g (0,1 m) Phenylethylamin versetzt und 1,5 Stunden gerührt. Dann fügt man 4,5 g Natriumboranat hinzu und läßt 1 Tag weiterrühren, engt ein und arbeitet mit Essigester/Wasser auf. Die Essigesterphase wird mit Wasser gewaschen, getrocknet und eingeengt. Den Rückstand löst man in Ether

und leitet trockenen Chlorwasserstoff ein. Der ausgefallene Niederschlag wird abgesaugt, mit Wasser verrührt und abgesaugt. Nach dem Trocknen wird aus Acetonnitril umkristallisiert. 20,5 g weiße Kristalle (62% der Theorie) vom Schmp. 180—3°C.

33) Herstellung nach Verfahrensvariante B

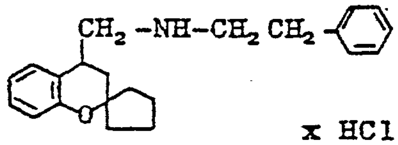

x HCl

12 g (0,1 m) Phenylacetaldehyd werden wie in oben stehendem Beispiel mit 21,7 g (0,1 m) 2-Spirocyclopenta-4-amino-methylchroman umgesetzt und aufgearbeitet. 19,5 g (55% der Theorie) weiße Kristalle vom Schmp. 202—3°.

34) Herstellung nach Verfahrensvariante B

Eine Lösung von 6,5 g 2,2-Spirocyclopenta-4-aminomethylchromen-(3) und 6,5 g 4-m-Trifluormethylphenyl-butanon-(2) in 30 ml Toluol wird 30 Minuten am Wasserabscheider zum Sieden erhitzt und anschließend bei 50°C/(10 torr) 1330 Pa eingeengt. Der ölige Rückstand wird in 50 ml Tetrahydrofuran gelöst und bei ca. 20°C zu einer Suspension von 5 g $LiA1H_4$ in 150 ml THF getropft. Man rührt 5 Stunden bei Rückflußtemperatur nach, kühlt auf 10°C und tropft nacheinander vorsichtig 5 ml Wasser und 15 ml 15 %ige Kalilauge zu, rührt 1 Stunde bei 20° nach und saugt ab. Das Filtrat wird eingeengt und destilliert. Ausbeute: 6,6 g fast farbloses ö1, Sdp. 200—210°C/(0,03 mmHg) 4 Pa.

35) Herstellung nach Verfahrensvariante C

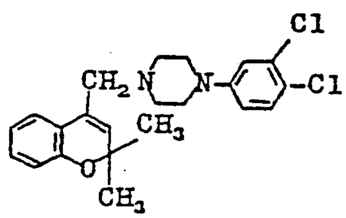

25 g (0,11 m) N-3,4-Dichlorphenyl-piperazin und 11,1 g (0,11 m) Triethylamin werden zusammen in 55 ml Toluol vorgelegt und mit 27,8 g (0,11 m) 2,2-Dimethyl-4-brom-methylchromen-3 tropfenweise versetzt. Dabei fällt ein Niederschlag aus, und die Temperatur steigt auf etwa 45°C. Man läßt 3 Stunden nachrühren und über Nacht stehen. Dann verrührt man gründlich mit überschüssiger 2n-Natronlauge, trennt die Toluolphase ab, wäscht sie mit Wasser und trocknet mit Natriumsulfat. Nach dem Abdestillieren des Lösungsmittels beginnt das Produkt auszufallen. Die Kristallisation wird durch Versetzen mit Petrolether vervollständigt. Nach Absaugen und Trocknen erhält man 27,5 g (62% der Theorie) weiße Kristalle vom Schmelzpunkt 108°C.

Auf ähnliche Weise wurden hergestellt;

36) nach Verfahren A und B

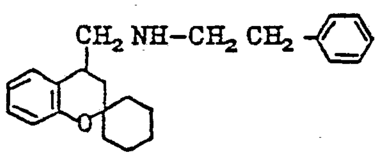

Kp. 175–8°C /(0,2 mmHg) 27 Pa

37) nach Verfahren A und B

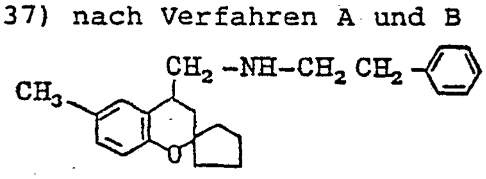

Schmp. des Hydrochlorids

253–6°C

38) nach Verfahren A und B

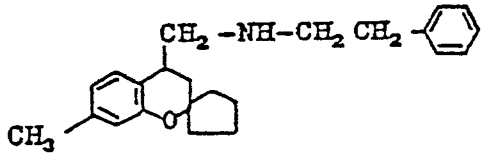

Schmp. des Hydrochlorids

245–8°C

39) nach Verfahren A und B

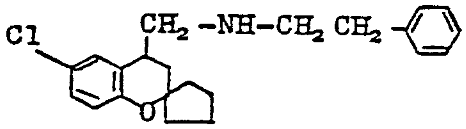

Kp. 180–5°C/(0,15 mmHg) 20 Pa

40) nach Verfahren A und B

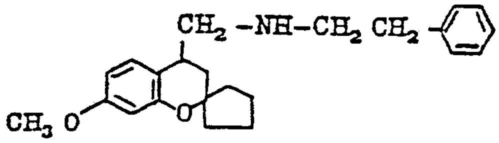

Schmp. des Hydrochlorids

220–3°C

41) nach Verfahren A und B

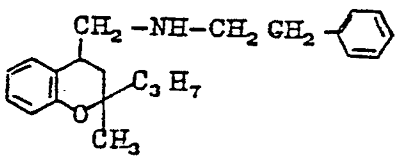

Kp. 175–9°C /(0,25 mmHg) 33 Pa

42) nach Verfahren A und B

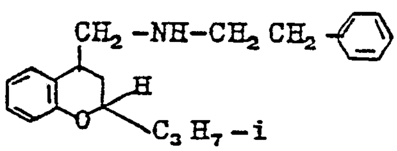

Kp. 172–7°C /(0,25 mmHg) 33 Pa

43) nach Verfahren A und B

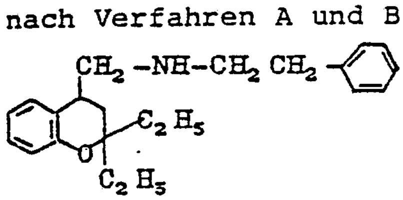

Kp. 190–5°C /(0,65 mmHg) 86 Pa

44) nach Verfahren A und B

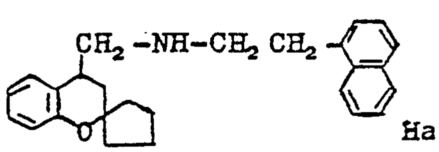

Harz

45) nach Verfahren A und B

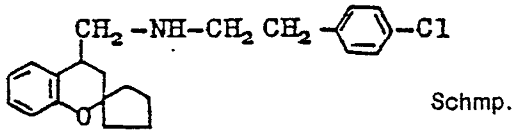

Schmp. des Hydrochlorids

180–2°C

46) nach Verfahren A und B

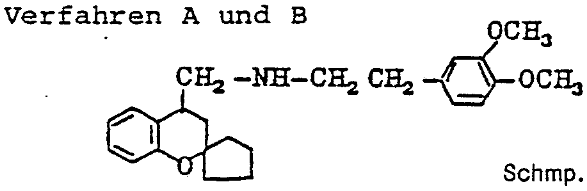

Schmp. des Hydrochlorids

198–200°C

47) nach Verfahren A und B

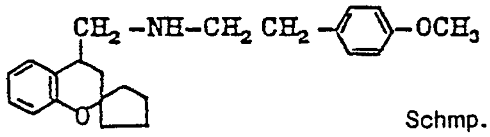

Schmp. des Hydrochlorids

214–6°C

48) nach Verfahren A und B

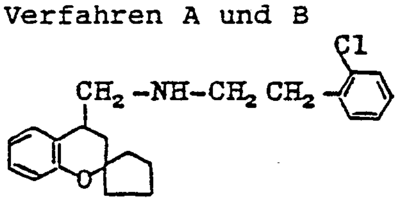

Schmp. des Hydrochlorids

212°C

49) nach Verfahren A und B

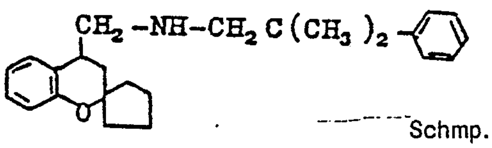

Schmp. des Hydrochlorids

190°C

50) nach Verfahren A und B

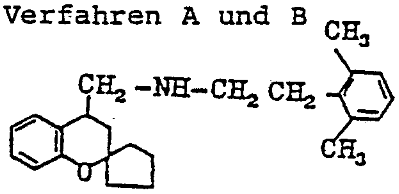

Schmp. des Hydrochlorids

231°C

51) nach Verfahren A und B

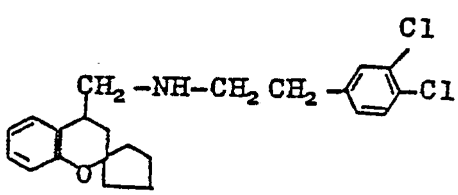

Kp. 198–203°C /(0,1 mmHg) 13 Pa

52) nach Verfahren A und B

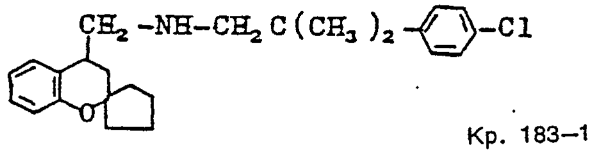

Kp. 183–190°C /(0,15 mmHg) 20 Pa

53) nach Verfahren A und B

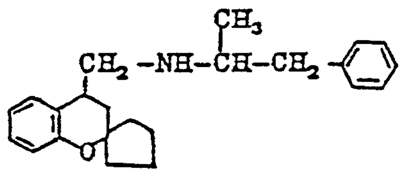

Kp. 171–7°C /(0,15 mmHg) 20 Pa

54) nach Verfahren A und B

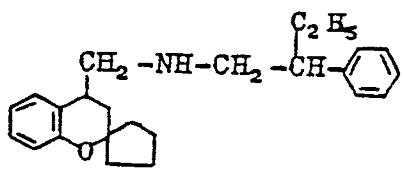

Kp. 184–9°C /(0,4 mmHg) 53 Pa

55) nach Verfahren A und B

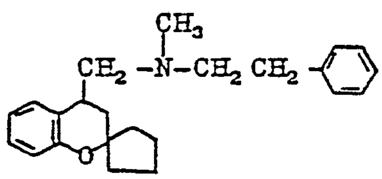

Kp. 168–75°C /(0,25 mmHg) 33 Pa

56) nach Verfahren A und B

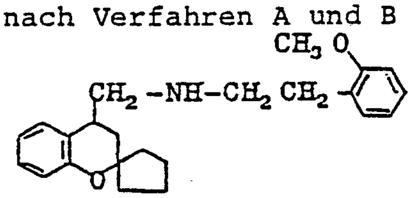

Schmp. des Hydrochlorids

208–10°C

57) nach Verfahren A und B

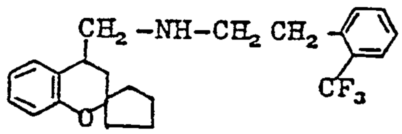

Kp. 185–95°C /(0,1 mmHg) 13 Pa

58) nach Verfahren A und B

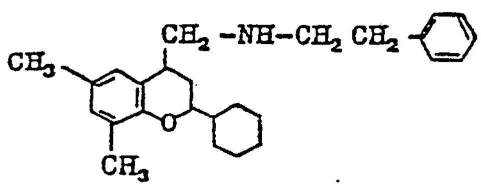

Kp. 188–94°C /(0,15 mmHg) 20 Pa

59) nach Verfahren A und B

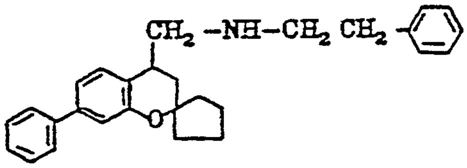

Harz

60) nach Verfahren A

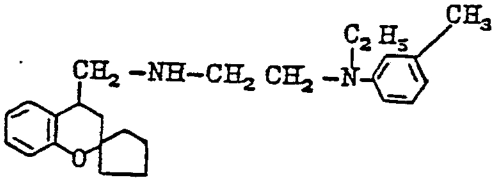

Kp. 190–6°C /(0,2 mmHg) 27 Pa

61) nach Verfahren A

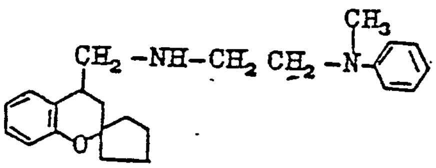

Kp. 181–3°C /(0,15 mmHg) 20 Pa

62) nach Verfahren A

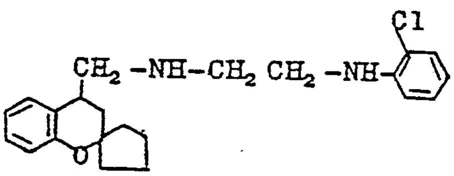

Kp. 195–202°C /(0,25 mmHg) 33 Pa

63) nach Verfahren A

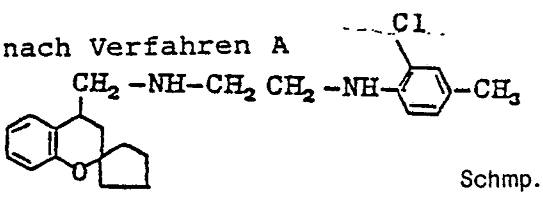

Schmp. des Hydrochlorids

170–2°C

64) nach Verfahren A

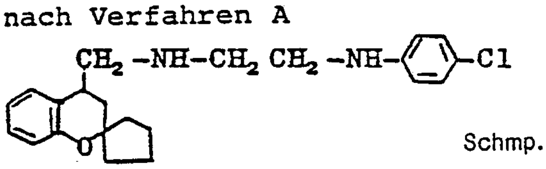

Schmp. des Hydrochlorids

168–70°C

65) nach Verfahren A

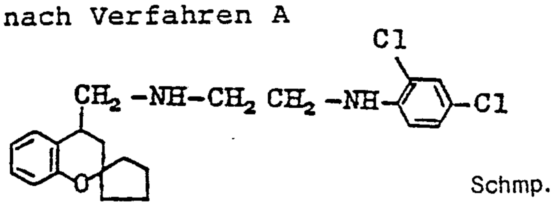

Schmp. des Hydrochlorids

185°C

66) nach Verfahren A

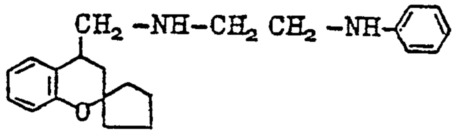

Schmp. des Hydrochlorids

202–4°C

67) nach Verfahren A und B

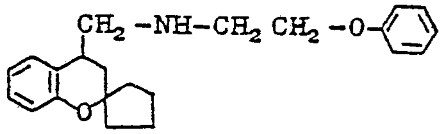

Kp. 178–82°C /(0,15 mmHg) 20 Pa

68) nach Verfahren A und B

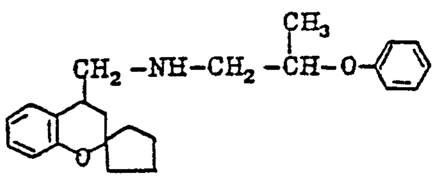

Kp. 185–7°C /(0,2 mmHg) 27 Pa

69) nach Verfahren A und B

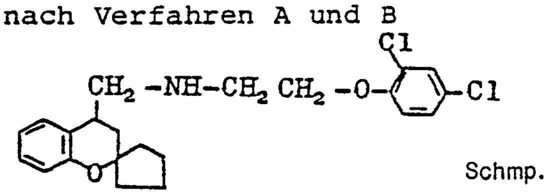

Schmp. des Hydrochlorids

185–7°C

70) nach Verfahren A und B

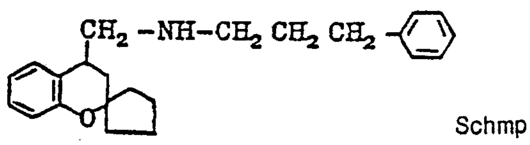

Schmp. des Hydrochlorids

102–4°C

71) nach Verfahren A und B

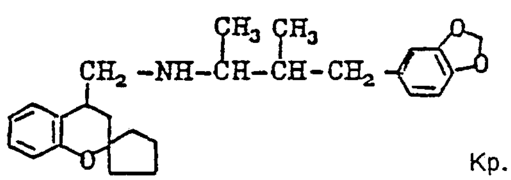

Kp. 198–205°C /(0,15 mmHg) 20 Pa

72) nach Verfahren A und B

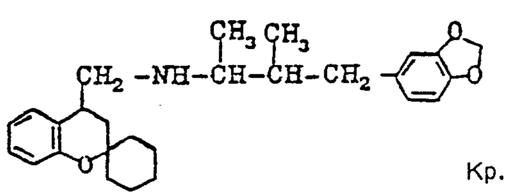

Kp. 207–15°C /(0,15 mmHg) 20 Pa

73) nach Verfahren C

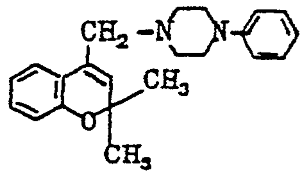

Schmp. 93–4°C

74) nach Verfahren C

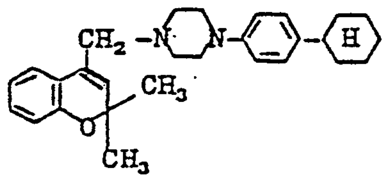

Schmp. 120–1°C

75) nach Verfahren C

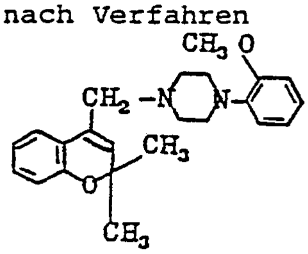

Schmp. 94–6°C

76) nach Verfahren C

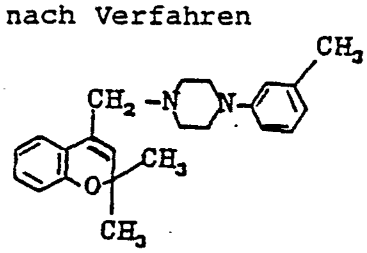

Schmp. 92–3°C

77) nach Verfahren C

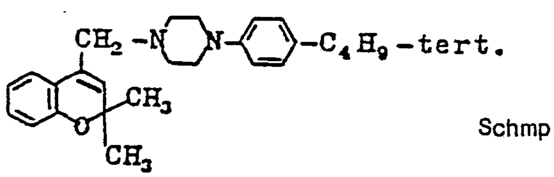

Schmp. 67–9°C

78) nach Verfahren C

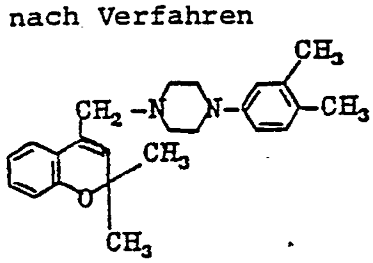

Schmp. 88–9°C

79) nach Verfahren C

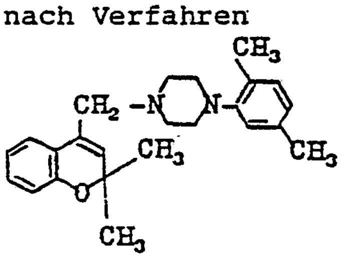

Kp. 193–9°C /(0,3 mmHg) 40 Pa

80) nach Verfahren C

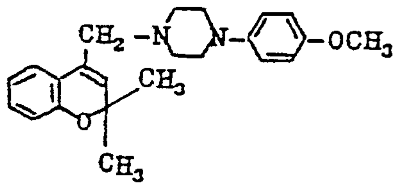

Schmp. 88°C

81) nach Verfahren C

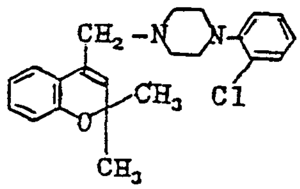

Schmp. 72°C

82) nach Verfahren C

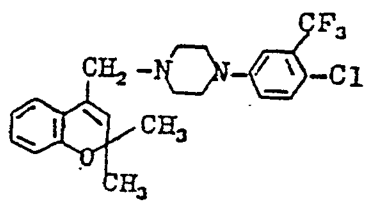

Schmp. 104–5°C

83) nach Verfahren C

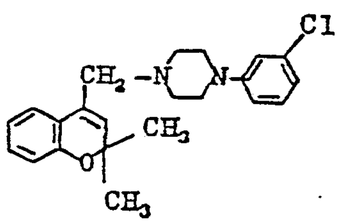

Schmp. 77°C

84) nach Verfahren C

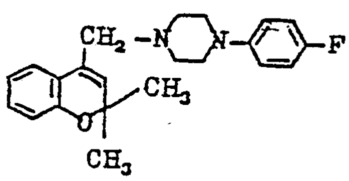

Schmp. 146°C

85) nach Verfahren C

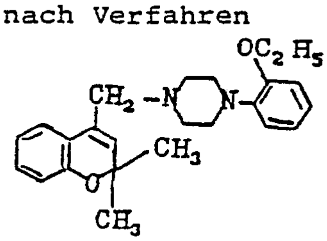

Schmp. 83°C

86) nach Verfahren C

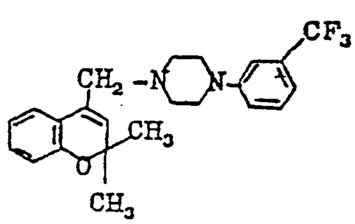

Kp. 192–8°C /(0,15 mmHg) 20 Pa

87) nach Verfahren C

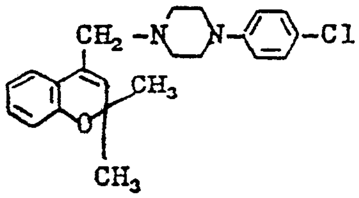

Schmp. 121°C

88) nach Verfahren C

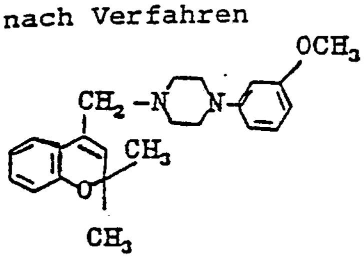

Schmp. 84°C

89) nach Verfahren C

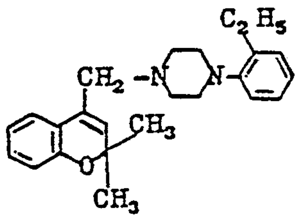

Kp. 189–94°C /(0,1 mmHg) 13 Pa

90) nach Verfahren C

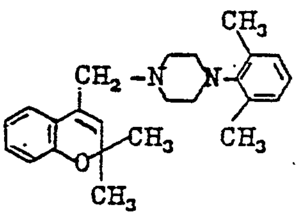

Kp. 191–9°C /(0,2 mmHg) 27 Pa

91) nach Verfahren C

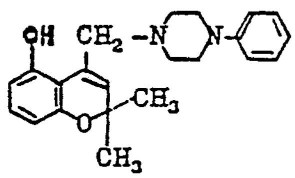

Schmp. 170–1°C

92) nach Verfahren A

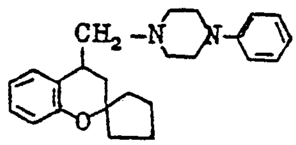

Kp. 180–4°C /(0,15 mmHg) 20 Pa

93) nach Verfahren C

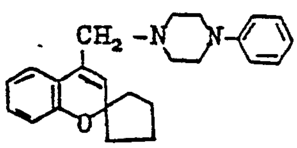

Schmp. 117–9°C

94) nach Verfahren C

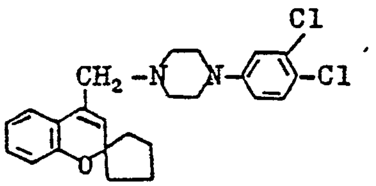

Schmp. 118–20°C

95) nach Verfahren C

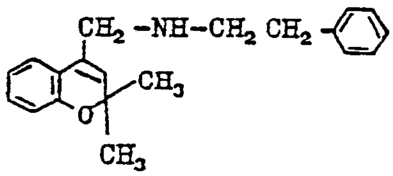

Kp. 198–203°C /(0,8 mmHg) 107 Pa

96) nach Verfahren B und C

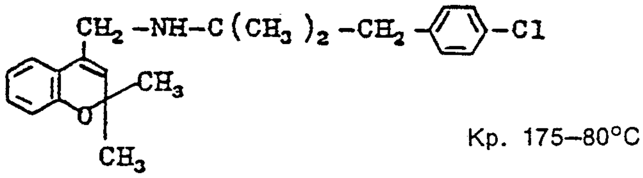

Kp. 175–80°C /(0,2 mmHg) 27 Pa

97) nach Verfahren A und B

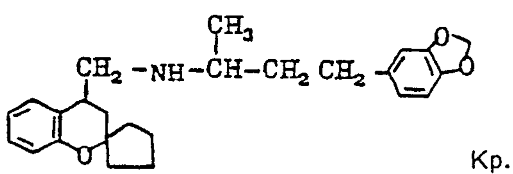

Kp. 191–6°C /(0,1 mmHg) 13 Pa

98) nach Verfahren A und B

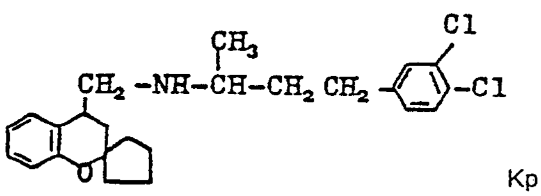

Kp. 201–212°C /(0,2 mmHg) 27 Pa

99) nach Verfahren A und B

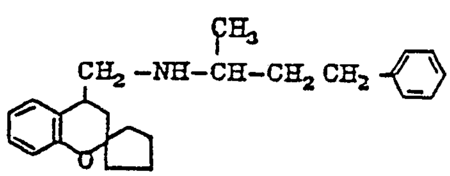

Kp. 182–7°C /(0,2 mmHg) 27 Pa

100) nach Verfahren A und B

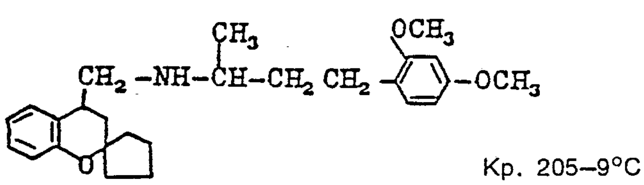

Kp. 205–9°C /(0,1 mmHg) 13 Pa

101) nach Verfahren A und B

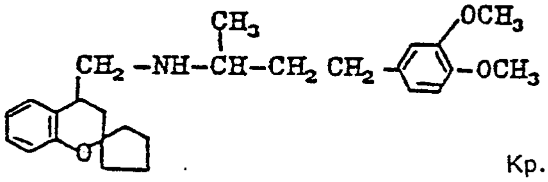

Kp. 202–10°C /(0,15 mmHg) 20 Pa

102) nach Verfahren A und B

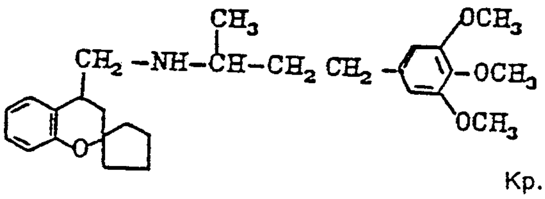

Kp. 204–12°C /(0,1 mmHg) 13 Pa

103) nach Verfahren A und B

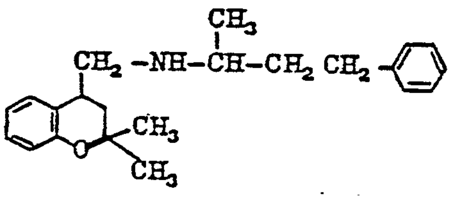

Kp. 171–4°C /(0,1 mmHg) 13 Pa

104) nach Verfahren A und B

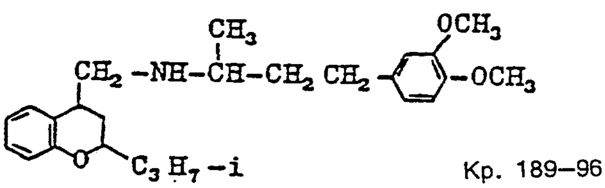

Kp. 189–96°C /(0,1 mmHg) 13 Pa

105) nach Verfahren A und B

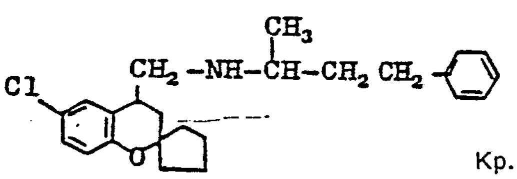

Kp. 190–8°C /(0,2 mmHg) 27 Pa

106) nach Verfahren A und B

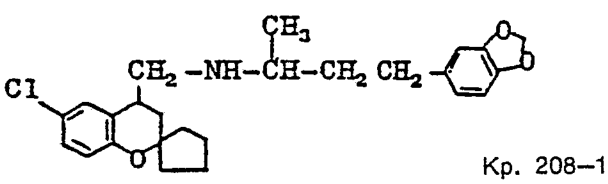

Kp. 208–15°C /(0,1 mmHg) 13 Pa

107) nach Verfahren A und B

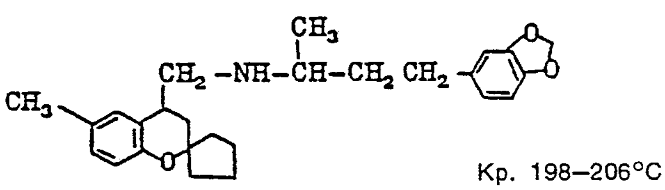

Kp. 198–206°C /(0,1 mmJg) 13 Pa

108) nach Verfahren A und B

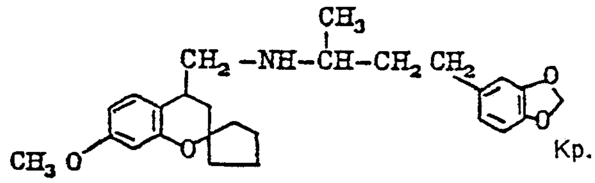

Kp. 205–12°C /(0,1 mmHg) 13 Pa

109) nach Verfahren A und B

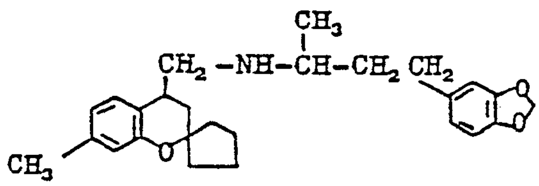

Kp. 200–5°C /(0,15 mmHg) 20 Pa

110) nach Verfahren A und B

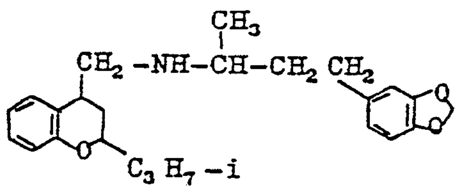

Kp. 185–92°C /(0,1 mmHg) 13 Pa

111) nach Verfahren A und B

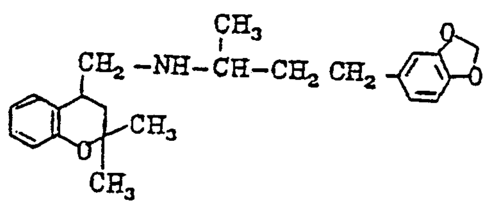

Kp. 181–7°C /0,1 mmHg) 13 Pa

112) nach Verfahren A und B

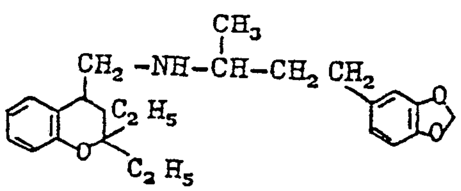

Kp. 198–204°C /(0,15 mmHg) 20 Pa

113) nach Verfahren A und B

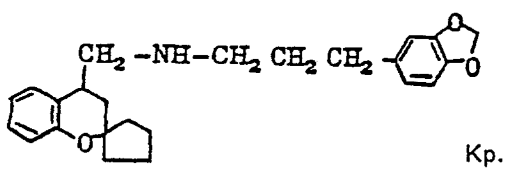

Kp. 190–2°C /(0,15 mmHg) 20 Pa

114) nach Verfahren A und B

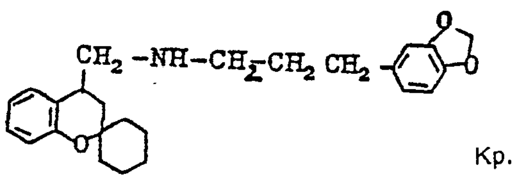

Kp. 199–204°C /(0,1 mmHg) 13 Pa

115) nach Verfahren B und C

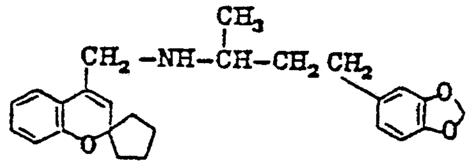

Kp. 210–215°C /(0,1 mmHg) 13 Pa

116) nach Verfahren A und B

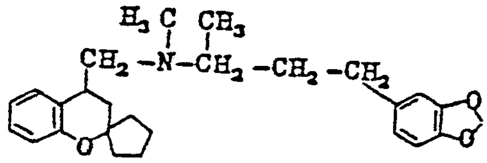

Kp. 215–8°C /(0,1 mmHg) 13 Pa

117) nach Verfahren B und C

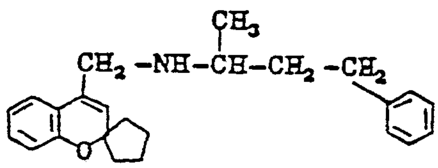

Kp. 200–205°C /(0,1 mmHg) 13 Pa

118) nach Verfahren A und B

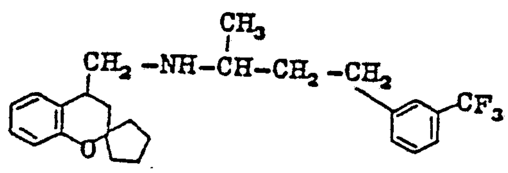

Kp. 195–200°C /(0,05 mmHg) 7 Pa

119) nach Verfahren B und C

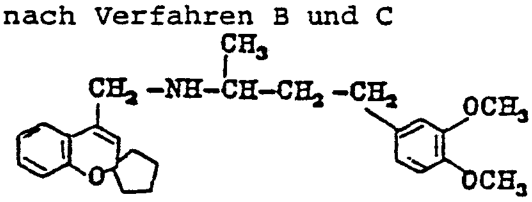

Kp. 220°C /(0,12 mmHg) 16 Pa

120) nach Verfahren A und B

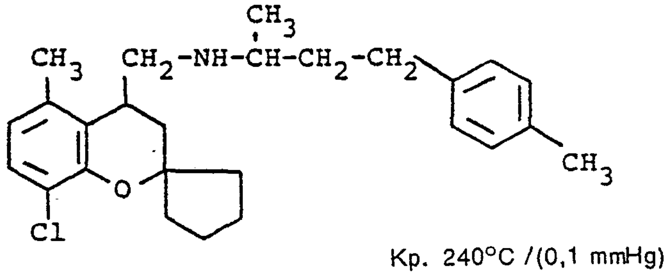

Kp. 240°C /(0,1 mmHg) 13 Pa

Herstellungsbeispiele für Chroman-4-carbaldehyde (Ausgangsprodukt für die Herstellungsvariante A)

Beispiel 121

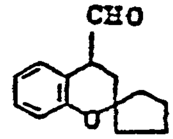

In einem 31-VA-Autoklaven wird eine Mischung aus 12 g Rh $[(C_6H_5)_3P]Cl_3$, 96 g Triphenylphosphin und 660 ml Toluol vorgelagt und bei 160—70°C Innentemperatur und einem $CO/H_2$-Druck von anfangs 150, später 300 bar langsam im Verlauf von 6 Stunden eine Mischung aus 665 g (3,58 m) 2-Spirocyclopentachromen-(3) zugepumpt. 1 Stunde nachrühren bei 170°C. Nach dem Entspannen wird aufdestilliert. Man erhält 327 g (42% der Theorie) 2-Spirocyclopentachroman-4-carbaldehyd als fast farblose Flüssigkeit vom Kp. 127—30°C/(0,1 mmHg) 13 Pa.

Beispiel 122

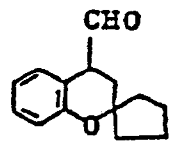

0,083 g Rh $[(C_6H_5—CH_2)_2S]_3Cl_3$ und 500 ml Toluol werden in einem Autoklaven 1h bei 170° und 170 bar $CO/H_2$-Druck gerührt. Man läßt auf 135° abkühlen und pumpt innerhalb 3h 558 g (3 m) 2-Spirocyclopentachromen-(3), gelöst in 600 ml Toluol, ein, wobei ein Druck von 250—280 bar aufrecht erhalten wird. Nach Abkühlen, Entspannen und Entgasen wird das Lösungsmittel im Vakuum abgezogen und der flüssige Rückstand fraktioniert. 518 g (80% der Theorie) farblose Flüssigkeit vom Kp. 127—30°C/0,3 mmHg) 40 Pa.

Auf ähnliche Weise wie in Beispiel 122 beschrieben wurden hergestellt:

123) 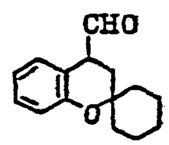 Kp. 120–5°C /(0,3 mmHg) 40 Pa

124) 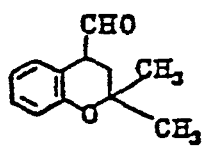 Kp. 90–95°C /(0,5 mmHg) 67 Pa

125) 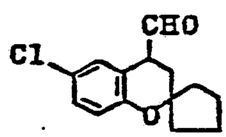 Kp. 120° C /(0,2 mmHg) 27 Pa

126) 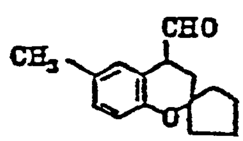 Kp. 106–10°C /(0,04 mmHg) 5 Pa

127) 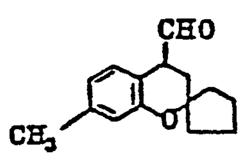 Kp. 121–6°C /(0,3 mmHg) 40 Pa

128) 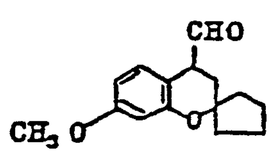 Kp. 143–30°C /(0,15 mmHg) 20 Pa

129) 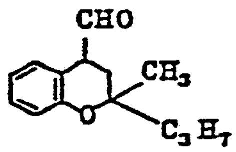 Kp. 110°C /(0,5 mmHg) 67 Pa

130) 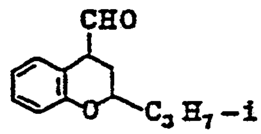 Kp. 110°C /(0,5 mmHg) 67 Pa

131) 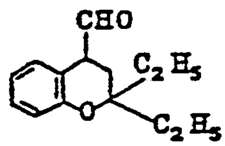 Kp. 110–5°C /(0,5 mmHg) 67 Pa

132) 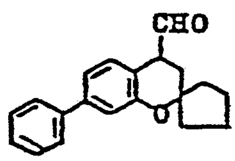 Kp. 165–70°C /(0,05 mmHg) 7 Pa

133) 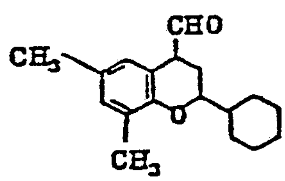 Kp. 110–115°C /(0,2 mmHg) 27 Pa

134) 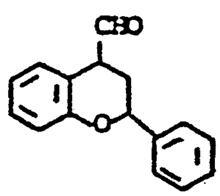 Kp. 145–50°C /(0,1 mmHg) 13 Pa

135) 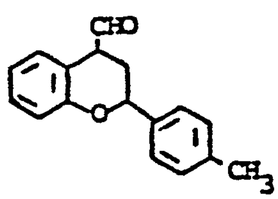 Kp. 152–157°C /(0,1 mmHg) 13 Pa

136) 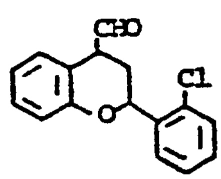 Kp. 161–3°C /(0,05 mmHg) 7 Pa

137) 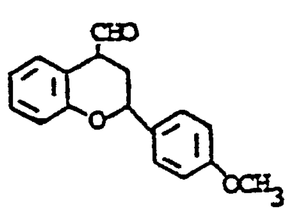 Kp. 155–162°C /(0,09 mmHg) 12 Pa

138 ) 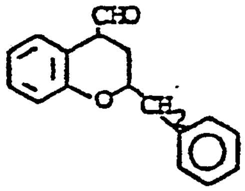 Kp. 154–61°C /(0,1 mmHg) 13 Pa

139 ) 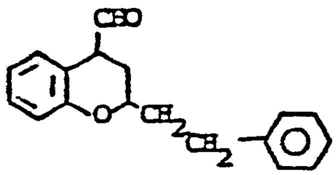 Kp. 152–9°C /(0,03 mmHg) 4 Pa

140 ) 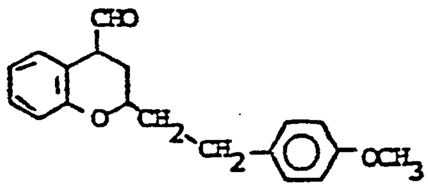 Kp. 159–66°C /(0,07 mmHg) 9 Pa

141 ) 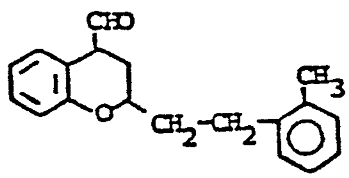 Kp. 163–9°C /(0,05 mmHg) 7 Pa

142 ) 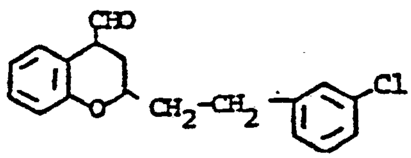 Kp. 170–8°C /(0,01 mmHg) 11 Pa

143 ) 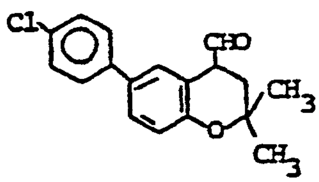 Kp. 173–5°C /(0,05 mmHg) 7 Pa

144 ) 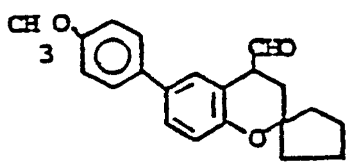 Kp. 182–9°C /(0,05 mmHg) 7 Pa

145 ) 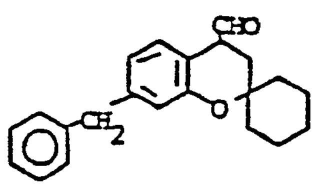 Kp. 180–6°C /(0,04 mmHg) 5 Pa

146 ) 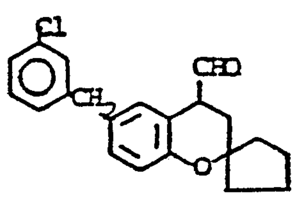 Kp. 189–93°C /(0,04 mmHg) 5 Pa

147) 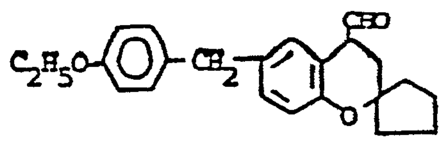 Kp. 188–98°C /(0,05 mmHg) 7 Pa

148) 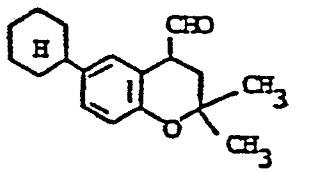 Kp. 160–6°C /(0,06 mmHg) 8 Pa

Beispiel 122 wurde unter Verwendung der folgenden Katalysatoren wiederholt:

a) $Co_2(CO)_8$
b) $[Rh(CO)_2Cl]_2$
c) $Rh(PPH_3)_3Cl$
d) $Rh(PPH_3)_3Cl$
e) $Rh[[C_2H_5)_2Fe]PPH_2]_3$ Fl
f) $RhCl_3 \times 3H_2O$
g) $Rh(C_8H_{14})_2Cl$
h) $Rh(CO)_2acac_3$
i) $HCo(CO)_3[P(Bu)_3]_3$
k) $Rh_4(CO)_{12}$
l) $Rn_3(CO)_{12}$
m) $Ir(CO)[PPH_3]_2Cl$

In allen Fällen wurde in guten Ausgeuten eine farblose Flüssigkeit von Kp. 127—30°C/(0,3 mm Hg) 40 Pa erhalten.

Prüfung der antihypertensiven wirkung an renal hypertonen Hunden Methode

Verwendet werden Beagle-Hunde beiderlei Geschlechts. Zur direkten Messung des Blutdrucks werden die Tiere mit einem Dauerkatheter in der Aorta versehen. Zur Erzielung eines experimentellen Hochdrucks wird die rechte A. renalis stenosiert. Systolischer und diastolischer Blutdruck sowie die Herzfrequenz werden kontinuierlich mit einem Funktelemetrie-System an wachen, frei beweglichen Hunden registriert. Die Daten werden durch ein on-line Datenakquisitionssystem (DAS 10/4; Fa. i.f.d.) über eine Sender/Empfänger-Kombination übernommen und durch einen programmierbaren Rechner (Multi 4; Fa. Intertechnique) ausgewertet. Die Daten werden als stündliche Mittel aller registrierten Blutdruckpulse ausgegeben.

Die Prüfsubstanzen werden in Polyethylenglykol 400 gelöst und in einer Gelatinekapsel gelöst oral verabreicht. Der Blutdruck wird während einer Vorperiode von einer Stunde, sowie bis zu 16 Stunden nach Gabe gemessen.

Als Grenzdosis wird die kleinste Dosis bezeichnet, mit der mindestens 15 mmHg Blutdrucksenkung erreicht werden.

Ergebnisse

| Substanz | Grenzdosis (mg/kg p.o.) | % Blutdrucksenkung |
|---|---|---|
| Beispiel 97 | 3,15 | 10 |
| Beispiel 102 | 1,00 | 9 |
| Beispiel 118 | 10,00 | 16 |

Prüfung der antihypertensiven Wirkung an spontan hypertonen Ratten:

Methode

Der Blutdruck wird am Schwanz der wachen Ratte unblutig mittels aufblasbarer Manschette und Infraton-Pulsabnehmer D nach BOUCKE-BRECHT gemessen. Die Tiere befinden sich während des ganzen Versuchs, einschließlich einer zweistündigen Vorperiode, einzeln in mit einem Wassermantel umgebenen, auf ca 30°C temperierten Kunststoffröhren. Die aufblasbare Gummimanschette sitzt an der Schwanzwurzel,

der Pulsabnehmer 3 cm distal davon. Zur Erzielung gleichmäßiger Meßresultate ist für die wiederholten Messungen die Meßstelle am Schwanz mit Tusche markiert.

Gemessen wird bei abfallendem Manschettendruck, wobei der in der Manschette herrschende Druck bei Wiederaufreten der Pulsationen dem systolischen Blutdruck in Rattenschwanz entspricht. Die Substanzen werden als Suspension mittels Schlundsonde oral apliziert. Vor Gabe und 1, 2, 4, 6, und 24 Stunden nach Gabe der Substanzen wird der systolische Blutdruck gemessen. Jede Dosis wird bei mindestens 3 Tieren untersucht. An jedem Versuchstag läuft eine unbehandelte Gruppe als Kontrolle parallel. Als Grenzdosis wird die kleinste Dosis bezeichnet. mit der mindestens 15 mm Hg Blutdrucksenkung erreicht werden.

| Substanz | Grenzdosis (mg/kg p.o.) |
|---|---|
| Beispiel 97 | 10,0 |
| Beispiel 102 | 1,0 |

**Patentansprüche**

1. Verbindungen der Formel (I)

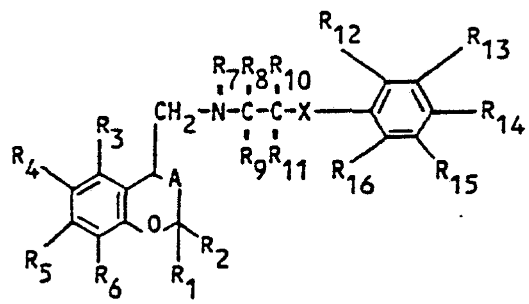

(I)

in der

—A— eine Einfachbindung oder eine Doppelbindung darstellt,

$R_1$ und $R_2$ gleich oder verschieden sind und für Wasserstoff, $C_1$—$C_6$-Alkyl, $C_5$—$C_7$-Cycloalkyl, Phenyl, das gegebenenfalls durch $C_1$—$C_4$-Alkyl, Halogen und/oder $C_1$—$C_4$-Alkoxy substituiert sein kann, sowie für $C_7$—$C_9$-Aralkyl, dessen Arylrest gegebenenfalls durch $C_1$—$C_4$-Alkyl, Halogen und/oder $C_1$—$C_4$-Alkoxy substituiert ist stehen,

oder $R_1$ und $R_2$ zusammen mit dem eingeschlossenen C-Atom des Chromanringes einen 4—7 gliedrigen carbocyclischen Ring bilden,

$R_3$ bis $R_6$ gleich oder verschieden sind und für Wasserstoff, Hydroxy, Halogen, $C_1$—$C_6$-Alkyl, $C_5$—$C_7$-Cycloalkyl, Phenyl, das gegebenenfalls durch $C_1$—$C_4$-Alkyl, Halogen und/oder $C_1$—$C_4$-Alkoxy substituiert ist, sowie für $C_7$—$C_9$-Aralkyl, dessen Arylrest gegebenenfalls durch $C_1$—$C_4$-Alkyl, Halogen und/oder $C_1$—$C_4$-Alkoxy substituiert ist, oder für $C_1$—$C_4$-Alkoxy stehen.

$R_7$, $R_8$, $R_9$, $R_{10}$, $R_{11}$ gleich oder verschieden sind und für Wasserstoff oder $C_1$—$C_6$-Alkyl stehen,

X für eine Einfachbindung, Methylen das gegebenenfalls ein- oder zweifach durch $C_1$—$C_4$-Alkyl Sauerstoff oder —$NR_{17}$ substituiert ist, steht, wobei $R_{17}$ Wasserstoff oder $C_1$—$C_4$-Alkyl darstellt oder $R_{17}$ zusammen mit $R_7$ einen $C_2$-Alkylenringschluß bildet, und

$R_{12}$, $R_{13}$, $R_{14}$, $R_{15}$, $R_{16}$ gleich oder verschieden sind und für Wasserstoff, Hydroxy, Halogen, $C_1$—$C_6$-Alkyl, $C_5$—C oder $C_6$-Cycloalkyl, Benzyl, $C_1$—$C_4$-Alkoxy, Trifluormethyl bedeuten, wobei $R_{12}$ und $R_{13}$ oder $R_{13}$ und $R_{14}$ zusammen jeweils eine $C_1$—$C_3$-Alkylendioxygruppe oder eine —CH=CH—CH=CH-Gruppe bilden können,

sowie ihre pharmazeutisch unbedenklichen Säureadditionssalze.

2. Verbindungen gemäß Formel (I) in Anspruch 1, in welcher

$R_1$ und $R_2$ gleich oder verschieden sind und Wasserstoff, $C_1$—$C_4$-Alkyl oder zusammen mit dem von ihnen eingeschlossenen Kohlenstoffatom einen carbocyclischen $C_5$ oder $C_6$ Ring darstellen,

$R_3$ bis $R_6$ gleich oder verschieden sind und Wasserstoff, Hydroxy, $C_1$—$C_4$-Alkyl, $C_1$—$C_4$-Alkoxy oder Chlor bedeuten,

$R_7$ bis $R_{11}$ gleich oder verschieden sind und Wasserstoff oder $C_1$—$C_4$-Alkyl darstellen,

X eine Einfachbindung, Sauerstoff, Methylen oder —$NR_{17}$ darstellt, wobei $R_{17}$ Wasserstoff oder $C_1$—$C_3$-Alkyl bedeutet, oder $R_{17}$ zusammen mit $R_7$ einen Ethylenringschluß bildet, und

$R_{12}$ bis $R_{16}$ gleich oder verschieden sind und Wasserstoff, Chlor, Cyclohexyl, $C_1$—$C_4$-Alkyl, $C_1$—$C_3$-Alkoxy, Trifluormethyl bedeuten, oder $R_{12}$ und $R_{13}$ oder $R_{13}$ und $R_{14}$ zusammen jeweils eine Methylendioxygruppe oder —CH=CH—CH=CH— bilden.

3. 4-[δ-(3,4-Methylendioxyphenyl)-α-methyl]-aminomethyl-2-spirocyclopentachroman.

4. 4-(β-Phenylethyl)-aminomethyl-2-spirocyclopentachroman.

5. 4-N-3,4-Dichlorphenyl-piperazin-N'-yl-methyl-2,2-dimethylchromen.

6. 4-β-Phenoxyethyl-aminomethyl-2-spirocyclopentachroman.

7. Verfahren zur Herstellung der Verbindungen der allgemeinen Formel (I)

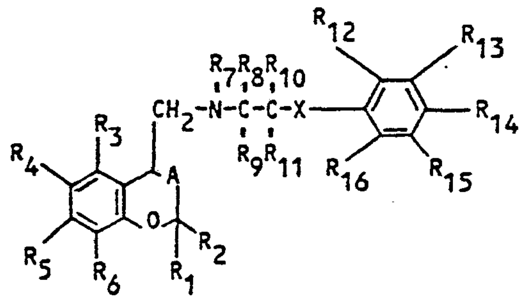

(I)

in der

—A— eine Einfachbindung oder eine Doppelbindung darstellt,

$R_1$ und $R_2$ gleich oder verschieden sind und für Wasserstoff, $C_1$—$C_6$-Alkyl, $C_5$—$C_7$-Cycloalkyl, Phenyl, das gegebenenfalls durch $C_1$—$C_4$-Alkyl, Halogen und/oder $C_1$—$C_4$-Alkoxy substituiert sein kann, sowie für $C_7$—$C_9$-Aralkyl, dessen Arylrest gegebenenfalls durch $C_1$—$C_4$-Alkyl, Halogen und/oder $C_1$—$C_4$-Alkoxy substituiert ist stehen,

oder $R_1$ und $R_2$ zusammen mit dem eingeschlossenen C-Atom des Chromanringes einen 4—7 gliedrigen carbocyclischen Ring bilden,

$R_3$ bis $R_6$ gleich oder verschieden sind und für Wasserstoff, Hydroxy, Halogen, $C_1$—$C_6$-Alkyl, $C_5$—$C_7$-Cycloalkyl, Phenyl, das gegebenenfalls durch $C_1$—$C_4$-Alkyl, Halogen und/oder $C_1$—$C_4$-Alkoxy substituiert ist, sowie für $C_7$—$C_9$-Aralkyl, dessen Arylrest gegebenenfalls durch $C_1$—$C_4$-Alkyl, Halogen und/oder $C_1$—$C_4$-Alkoxy substituiert ist, oder für $C_1$—$C_4$-Alkoxy stehen.

$R_7$, $R_8$, $R_9$, $R_{10}$, $R_{11}$ gleich oder verschieden sind und für Wasserstoff oder $C_1$—$C_6$-Alkyl stehen,

X für eine Einfachbindung, Methylen das gegebenenfalls ein- oder zweifach durch $C_1$—$C_4$-Alkyl, Sauerstoff oder —$NR_{17}$ substituiert ist, steht, wobei $R_{17}$ Wasserstoff oder $C_1$—$C_4$-Alkyl darstellt oder $R_{17}$ zusammen mit $R_7$ einen $C_2$-Alkylenringschluß bildet, und

$R_{12}$, $R_{13}$, $R_{14}$, $R_{15}$, $R_{16}$ gleich oder verschieden sind und für Wasserstoff, Hydroxy, Halogen, $C_1$—$C_6$-Alkyl, $C_5$- oder $C_6$-Cycloalkyl, Benzyl, $C_1$—$C_4$-Alkoxy, Trifluormethyl bedeuten, wobei $R_{12}$ und $R_{13}$ oder $R_{13}$ und $R_{14}$ zusammen jeweils eine $C_1$—$C_3$-Alkylendioxygruppe oder eine —CH=CH—CH=CH-Gruppe bilden können, dadurch gekennzeichnet, daß man entweder

A) Chroman-4-carbaldehyde der Formel (II)

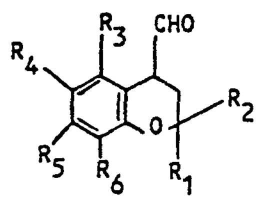

(II)

in welcher

$R_1$, $R_2$, $R_3$, $R_4$, $R_5$ und $R_6$ die oben angegebenen Bedeutung haben, mit Aminen der Formel (III)

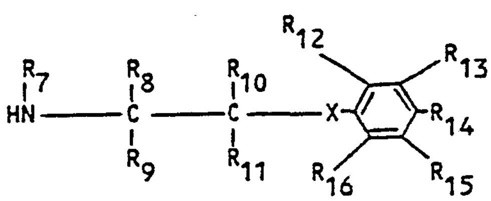

(III)

in welcher

$R_7$, $R_8$, $R_9$, $R_{10}$, $R_{11}$, $R_{12}$, $R_{13}$, $R_{14}$, $R_{15}$, $R_{16}$, $R_{17}$ und X die oben angegebene Bedeutung haben, in Gegenwart von reduzierenden Agentien umsetzt, oder

B) Amine der Formel (IV)

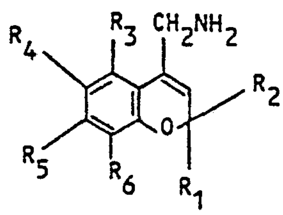

(IV)

oder

der Formel (V)

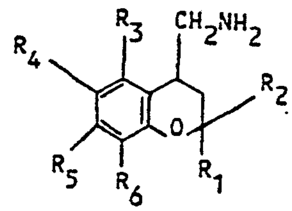

(V)

in welchen

$R_1$, $R_2$, $R_3$, $R_4$, $R_5$ und $R_6$ die oben angegebene Bedeutung haben, mit Carbonylverbindungen der Formel (VI)

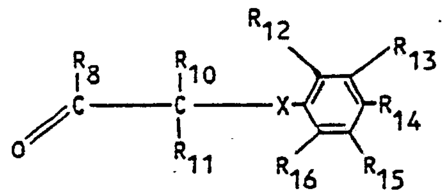

(VI)

in welcher

$R_8$—$R_{16}$ die oben angegebene Bedeutung haben, mit der Maßgabe, daß X nicht für $NR_{17}$ steht, in Gegenwart von reduzierenden Agentien umsetzt, oder

B) Verbindungen der Formel (VII)

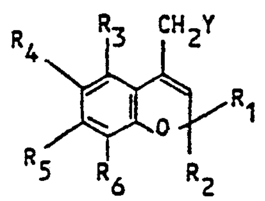

(VII)

in welcher

$R_1$, $R_2$, $R_3$, $R_4$, $R_5$ und $R_6$ die oben angegebene Bedeutung haben, und

Y für Brom oder Chlor steht,

mit den Aminen der Formel (III) in Gegenwart eines säurebindenden Mittels umsetzt.

8. Verbindungen gemäß Ansprüchen 1—6 zur Verwendung bei der Bekämpfung von Kreislauferkrankungen.

9. Arzneimittel enthaltend als Wirkstoff ein oder mehrere Verbindungen gemäß Ansprüchen 1—6.

10. Verfahren zur Herstellung von Arzneimitteln gemäß Anspruch 9, dadurch gekennzeichnet, daß man die Verbindungen gemäß Ansprüchen 1—6 mit inerten, nicht-toxischen pharmazeutisch geeigneten Trägerstoffen oder Lösungsmitteln vermischt.

11. Verwendung der Verbindungen gemäß Ansprüchen 1—6 bei der Herstellung von kreislaufwirksamen Arzneimitteln.

**Claims**

1. Compounds of the formula (I)

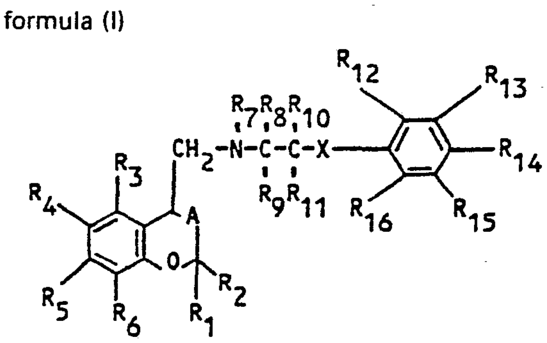

(I)

in which

—A— represents a single bond or a double bond,

$R_1$ and $R_2$ are identical or different and represent hydrogen, $C_1$—$C_6$-alkyl, $C_5$—$C_7$-cycloalkyl, phenyl which can optionally be substituted by $C_1$—$C_4$-alkyl, halogen and/or $C_1$—$C_4$-alkoxy, and $C_7$—$C_9$-aralkyl, the aryl radical of which is optionally substituted by $C_1$—$C_4$-alkyl, halogen and/or $C_1$—$C_4$-alkoxy, or

$R_1$ and $R_2$, together with the enclosed C atom of the chroman ring, form a 4—7-membered carbocyclic ring,

$R_3$ to $R_6$ are identical or different and represent hydrogen, hydroxyl, halogen, $C_1$—$C_6$-alkyl, $C_5$—$C_7$-cycloalkyl, phenyl which is optionally substituted by $C_1$—$C_4$-alkyl, halogen and/or $C_1$—$C_4$-alkoxy, and $C_7$—$C_9$-aralkyl, the aryl radical of which is optionally substituted by $C_1$—$C_4$-alkyl, halogen and/or $C_1$—$C_4$-alkoxy, or $C_1$—$C_4$-alkoxy,

$R_7$, $R_8$, $R_9$, $R_{10}$ and $R_{11}$ are identical or different and represent hydrogen or $C_1$—$C_6$-alkyl,

X represents a single bond, methylene which is optionally mono- or disubstituted by $C_1$—$C_4$-alkyl, oxygen or —$NR_{17}$, wherein

$R_{17}$ represents hydrogen or $C_1$—$C_4$-alkyl or

$R_{17}$ together with $R_7$, forms a $C_2$-alkylene ring-closing member, and

$R_{12}$, $R_{13}$, $R_{14}$, $R_{15}$ and $R_{16}$ are identical or different and denote hydrogen, hydroxyl, halogen, $C_1$—$C_6$-alkyl, $C_5$- or $C_6$-cycloalkyl, benzyl, $C_1$—$C_4$-alkoxy or trifluoromethyl, it being possible for $R_{12}$ and $R_{13}$ or $R_{13}$ and $R_{14}$ together in each case to form a $C_1$—$C_3$-alkylenedioxy group or a —CH=CH—CH=CH— group,

and their pharmaceutically harmless acid addition salts.

2. Compounds according to formula (I) in Claim 1, in which

$R_1$ and $R_2$ are identical or different and represent hydrogen, $C_1$—$C_4$-alkyl or together with the carbon atom which they enclose represent a carbocyclic $C_5$ or $C_6$ ring,

$R_3$ to $R_6$ are identical or different and denote hydrogen, hydroxyl, $C_1$—$C_4$-alkyl, $C_1$—$C_4$-alkoxy or chlorine,

$R_7$ to $R_{11}$ are identical or different and represent hydrogen or $C_1$—$C_4$-alkyl,

X represents a single bond, oxygen, methylene or —$NR_{17}$, wherein

$R_{17}$ denotes hydrogen or $C_1$—$C_3$-alkyl, or

$R_{17}$, together with $R_7$, forms an ethylene ring-closing member, and

$R_{12}$ to $R_{16}$ are identical or different and denote hydrogen, chlorine, cyclohexyl, $C_1$—$C_4$-alkyl, $C_1$—$C_3$-alkoxy, or trifluoromethyl, or $R_{12}$ and $R_{13}$ or $R_{13}$ and $R_{14}$ together in each case form a methylenedioxy group or —CH=CH—CH=CH—.

3. 4-[δ-(3,4-Methylenedioxyphenyl)-α-methyl]-aminomethyl-2-spirocyclopentachroman.

4. 4-(β-Phenylethyl)-aminomethyl-2-spirocyclopentachroman.

5. 4-N-3,4-Dichlorophenyl-piperazin-N'-yl-methyl-2,2-dimethylchromene.

6. 4-β-Phenoxyethyl-aminomethyl-2-spirocyclopentachroman.

7. Process for the preparation of the compounds of the general formula (I)

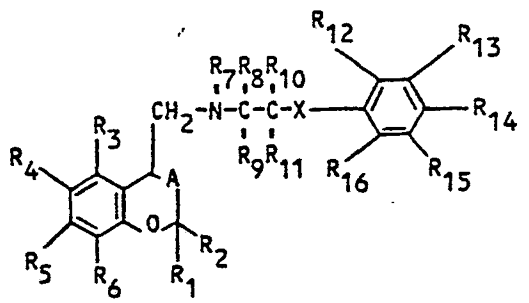

(I)

in which

—A— represents a single bond or a double bond,

$R_1$ and $R_2$ are identical or different and represent hydrogen, $C_1$—$C_6$-alkyl, $C_5$—$C_7$-cycloalkyl, phenyl which can optionally be substituted by $C_1$—$C_4$-alkyl, halogen and/or $C_1$—$C_4$-alkoxy, and $C_7$—$C_9$-aralkyl, the aryl radical of which is optionally substituted by $C_1$—$C_4$-alkyl, halogen and/or $C_1$—$C_4$-alkoxy, or

$R_1$ and $R_2$, together with the enclosed C atom of the chroman ring, form a 4—7-membered carbocyclic ring,

$R_3$ to $R_6$ are identical or different and represent hydrogen, hydroxyl, halogen, $C_1$—$C_6$-alkyl, $C_5$—$C_7$-cycloalkyl, phenyl which is optionally substituted by $C_1$—$C_4$-alkyl, halogen and/or $C_1$—$C_4$-alkoxy, and $C_7$—$C_9$-aralkyl, the aryl radical of which is optionally substituted by $C_1$—$C_4$-alkyl, halogen and/or $C_1$—$C_4$-alkoxy, or $C_1$—$C_4$-alkoxy,

$R_7$, $R_8$, $R_9$, $R_{10}$ and $R_{11}$ are identical or different and represent hydrogen or $C_1$—$C_6$-alkyl,

X represents a single bond, methylene which is optionally mono- or disubstituted by $C_1$—$C_4$-alkyl, oxygen or —$NR_{17}$, wherein

$R_{17}$ represents hydrogen or $C_1$—$C_4$-alkyl or

$R_{17}$ together with $R_7$, forms a $C_2$-alkylene ring-closing member, and

$R_{12}$, $R_{13}$, $R_{14}$, $R_{15}$ and $R_{16}$ are identical or different and denote hydrogen, hydroxyl, halogen, $C_1$—$C_6$-alkyl, $C_5$- or $C_6$-cycloalkyl, benzyl, $C_1$—$C_4$-alkoxy or trifluoromethyl, it being possible for $R_{12}$ and $R_{13}$ or $R_{13}$ and $R_{14}$ together in each case to form a $C_1$—$C_3$-alkylenedioxy group or a —CH=CH—CH=CH— group,

characterised in that either

A) chroman-4-carbaldehydes of the formula (II)

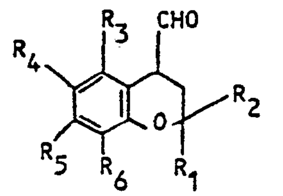

(II)

in which

$R_1$, $R_2$, $R_3$, $R_4$, $R_5$ and $R_6$ have the abovementioned meaning, are reacted with amines of the formula (III)

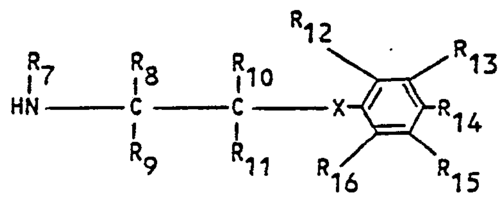

(III)

in which

$R_7$, $R_8$, $R_9$, $R_{10}$, $R_{11}$, $R_{12}$, $R_{13}$, $R_{14}$, $R_{15}$, $R_{16}$, $R_{17}$ and X have the abovementioned meaning, in the presence of reducing agents, or

B) amines of the formula (IV)

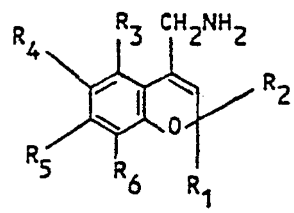

(IV)

or

of the formula (V)

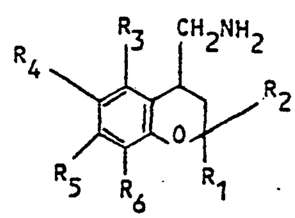

(V)

in which

$R_1$, $R_2$, $R_3$, $R_4$, $R_5$ and $R_6$ have the abovementioned meaning, are reacted with carbonyl compounds of the formula (VI)

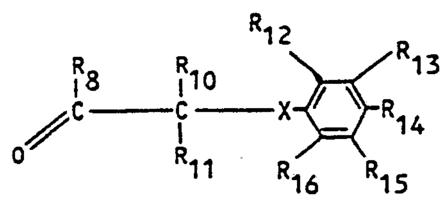

(VI)

in which

$R_8$—$R_{16}$ have the abovementioned meaning, with the proviso that X does not represent $NR_{17}$, in the presence of reducing agents, or

B) compounds of the formula (VII)

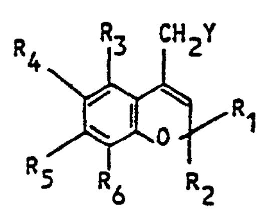

(VII)

in which

$R_1$, $R_2$, $R_3$, $R_4$, $R_5$ and $R_6$ have the abovementioned meaning and Y represents bromine or chlorine, are reacted with the amines of the formula (III) in the presence of an acid-binding agent.

8. Compounds according to Claims 1—6 for use in combating circulatory diseases.

9. Medicaments containing as the active compound one or more compounds according to Claims 1—6.

10. Process for the preparation of medicaments according to Claim 9, characterised in that the compounds according to Claims 1—6 are mixed with inert, non-toxic pharmaceutically suitable excipients or solvents.

11. Use of the compounds according to Claims 1—6 in the production of medicaments which act on the circulation.

**Revendications**

1. Composés de formule (I):

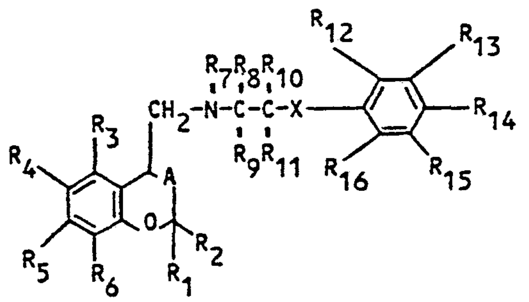

(I)

dans laquelle

—A— représente une liaison simple ou une liaison double,

$R_1$ et $R_2$ sont identiques ou différents et représentent chacun un atome d'hydrogène, un groupe alkyle en $C_1$ à $C_6$, cycloalkyle en $C_5$ à $C_7$, phényle (qui peut être éventuellement substitué par un groupe alkyle en $C_1$ à $C_4$, un atome d'halogène et/ou un groupe alcoxy en $C_1$ à $C_4$), ainsi qu'un groupe aralkyle en $C_7$ à $C_9$ (dont le reste aryle peut éventuellement être substitué par un groupe alkyle en $C_1$ à $C_4$, un halogène et/ou un groupe alcoxy en $C_1$ à $C_4$), ou bien $R_1$ et $R_2$ forment, avec l'atome de carbone inclus du noyau chromane, un noyau carbocyclique ayant 4 à 7 chaînons,

$R_3$ à $R_6$ sont identiques ou différents et représentent chacun un atome d'hydrogène, un groupe hydroxy, un atome d'halogène, un groupe alkyle en $C_1$ à $C_6$, cycloalkyle en $C_3$ à $C_7$, phényle (qui est éventuellement substitué par un groupe alkyle en $C_1$ à $C_4$, un atome d'halogène et/ou un groupe alcoxy en $C_1$ à $C_4$) ainsi qu'un groupe aralkyle en $C_7$ à $C_9$ (dont le reste aryle est éventuellement substitué par un groupe alkyle en $C_1$ à $C_4$, un atome d'halogène et/ou un groupe alcoxy en $C_1$ à $C_4$) ou un groupe alcoxy en $C_1$ à $C_4$,

$R_7$, $R_8$, $R_9$, $R_{10}$, $R_{11}$ sont identiques ou différents et représentent chacun un atome d'hydrogène ou un groupe alkyle en $C_1$ à $C_6$,

X représente une liaison simple, un groupe méthylène qui est éventuellement substitué une ou deux fois par un groupe alkyle en $C_1$ à $C_4$, un atome d'oxygène ou un groupe $—NR_{17}$, où $R_{17}$ représente un atome d'hydrogène ou un groupe alkyle en $C_1$ à $C_4$, ou bien $R_{17}$ forme avec $R_7$ un groupe alkylène en $C_2$ assurant une fermeture de cycle, et

$R_{12}$, $R_{13}$, $R_{14}$, $R_{15}$, $R_{16}$ sont identiques ou différents et représentent chacun un atome d'hydrogène, un groupe hydroxy, halogéno, alkyl en $C_1$ à $C_6$, cycloalkyle en $C_5$ ou $C_6$, benzyle, alcoxy en $C_1$ à $C_4$, trifluorométhyle, $R_{12}$ et $R_{13}$ ou bien $R_{13}$ et $R_{14}$ pouvant formés ensemble à chaque fois un groupe alkylènedioxy en $C_1$ à $C_3$ ou un groupe $—CH{=}CH—CH{=}CH—$,

ainsi que leurs sels d'addition d'acide acceptables du point de vue pharmaceutique.

2. Composés selon la formule (I) de la revendication 1, dans laquelle

$R_1$ et $R_2$ sont identiques ou différents et représentent chacun un atome d'hydrogène, un groupe alkyle en $C_1$ à $C_4$ ou bien, pris avec l'atome de carbone inclus entre eux, ils forment un noyau carbocyclique en $C_5$ ou $C_6$,

$R_3$ à $R_6$ sont identiques ou différents et représentent chacun un atome d'hydrogène, un groupe hydroxy, alkyle en $C_1$ à $C_4$, alcoxy en $C_1$ à $C_4$ ou un atome de chlore,

$R_7$ à $R_{11}$ sont identiques ou différents et représentent chacun un atome d'hydrogène ou un groupe alkyle en $C_1$ à $C_4$,

X représente une liaison simple, un atome d'oxygène, un groupe méthylène ou un $—NR_{17}$, où $R_{17}$ représente un atome d'hydrogène ou un groupe alkyle en $C_1$ à $C_3$, ou bien $R_{17}$ forme avec $R_7$ un groupe éthylène pour fermeture de cycle, et

$R_{12}$ à $R_{16}$ sont identiques ou différents et représentent chacun un atome d'hydrogène ou de chlore, un groupe cyclohexyle, alkyle en $C_1$ à $C_4$, alcoxy en $C_1$ à $C_3$, trifluorométhyle, ou bien $R_{12}$ et $R_{13}$ ou $R_{13}$ et $R_{14}$ forment ensemble à chaque fois un groupe méthylènedioxy ou $—CH{=}CH—CH{=}CH—$.

3. Le 4-[α-(3,4-méthylènedioxyphényl)-α-méthyl]-aminométhyl-2-spirocyclopentachromane.

4. Le 4-(β-phényléthyl)-aminométhyl-2-spirocyclopentachromane.

5. Le 4-N-3,4-dichlorophényl-pipérazin-N'-yl-méthyl-2,2-diméthylchromène.

6. Le 4-β-phénoxyéthyl-aminométhyl-2-spirocyclopentachromane.

7. Procédé pour produire des composés de formule générale (I):

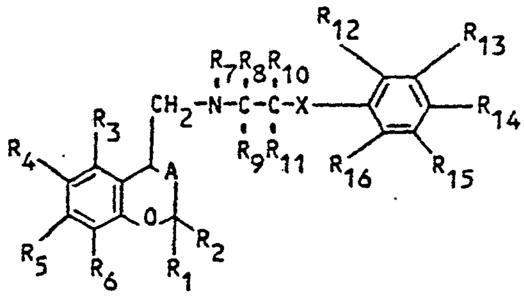

(I)

dans laquelle

—A— représente une liaison simple ou une liaison double,

$R_1$ et $R_2$ sont identiques ou différents et représentent chacun un atome d'hydrogène, un groupe alkyle en $C_1$ à $C_6$, cycloalkyle en $C_5$ à $C_7$, phényle (qui peut être éventuellement substitué par un groupe alkyle en $C_1$ à $C_4$, par de l'halogène et/ou par un groupe alcoxy en $C_1$ à $C_4$), ainsi qu'un groupe aralkyle en $C_7$ à $C_9$ (dont le reste aryle est éventuellement substitué par un groupe alkyle en $C_1$ à $C_4$, un atome d'halogène et/ou un groupe alcoxy en $C_1$ à $C_4$), ou bien $R_1$ et $R_2$ forment ensemble avec l'atome de carbone inclus du noyau chromane un noyau carbocyclique à 4 à 7 chaînons,

$R_3$ à $R_6$ sont identiques ou différents et représentent chacun un atome d'hydrogène, un groupe hydroxy, un atome d'halogène, un groupe alkyle en $C_1$ à $C_6$, cycloalkyle en $C_3$ à $C_7$, phényle (qui est éventuellement substitué par un groupe alkyle en $C_1$ à $C_4$, un atome d'halogène et/ou un groupe alcoxy en $C_1$ à $C_4$) ainsi qu'un groupe aralkyle en $C_7$ à $C_9$ (dont le reste aryle est éventuellement substitué par un groupe alkyle en $C_1$ à $C_4$, un atome d'halogène et/ou un groupe alcoxy en $C_1$ à $C_4$) ou un groupe alcoxy en $C_1$ à $C_4$,

$R_7$, $R_8$, $R_9$, $R_{10}$, $R_{11}$ sont identiques ou différents et représentent chacun un atome d'hydrogène ou un groupe alkyle en $C_1$ à $C_6$,

X représente une liaison simple, un groupe méthylène être qui est éventuellement où substitué une ou deux fois par un groupe alkyle en $C_1$ à $C_4$, un atome d'oxygène ou un groupe $—NR_{17}$, oú $R_{17}$ représente un atome d'hydrogène ou un groupe alkyle en $C_1$ à $C_4$, ou bien $R_{17}$ forme avec $R_7$ un groupe alkylène en $C_2$ pour fermeture de cycle, et

$R_{12}$, $R_{13}$, $R_{14}$, $R_{15}$, $R_{16}$ sont identiques ou différents et représentent chacun un atome d'hydrogène, un groupe hydroxy, un atome halogène, un groupe alkyl en $C_1$ à $C_6$, cycloalkyle en $C_5$ ou $C_6$, benzyle, alcoxy en $C_1$ à $C_4$, trifluorométhyle, $R_{12}$ et $R_{13}$ ou bien $R_{13}$ et $R_{14}$ pouvant formés ensemble à chaque fois un groupe alkylènedioxy en $C_1$ à $C_3$ ou un groupe $—CH=CH—CH=CH—$,

caractérisé en ce que:

A) on fait réagir des chromane-4-carbaldéhydes de formule (II)

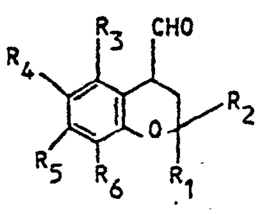

(II)

dans laquelle

$R_1$, $R_2$, $R_3$, $R_4$, $R_5$ et $R_6$ ont le sens indiqué ci-dessus avec des amines de formule (III):

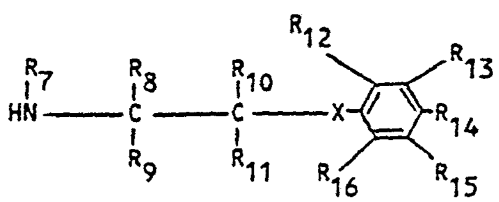

(III)

dans laquelle

$R_7$, $R_8$, $R_9$, $R_{10}$, $R_{11}$, $R_{12}$, $R_{13}$, $R_{14}$, $R_{15}$, $R_{16}$, $R_{17}$ et X ont le sens indiqué ci-dessus,
en présence d'agents réducteurs, ou

B) on fait réagir des amines de formule (IV):

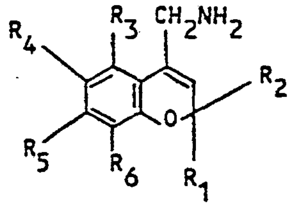

(IV)

ou

de formule (V):

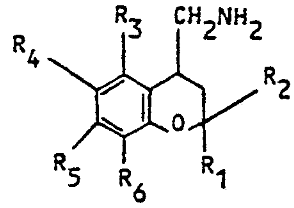

(V)

dans lesquelles

$R_1$, $R_2$, $R_3$, $R_4$, $R_5$ et $R_6$ ont le sens indiqué ci-dessus, avec des composés carbonylés de formule (VI):

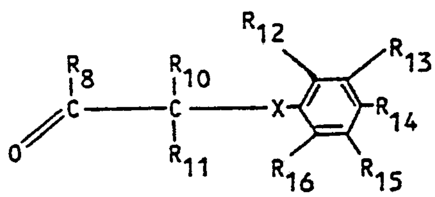

(VI)

dans laquelle

$R_8$ à $R_{16}$ ont le sens indiqué ci-dessus, à la condition que X ne représente pas $NR_{17}$, en présence d'agents réducteurs, ou

C) on fait réagir des composés de formule (VII):

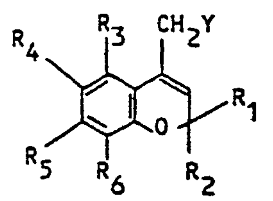

(VII)

dans laquelle

$R_1$, $R_2$, $R_3$, $R_4$, $R_5$ et $R_6$ ont le sens indiqué ci-dessus, et

Y représente un atome de brome ou de chlore, avec les amines de formule (III), en présence d'un agent de fixation des acides.

8. Composés selon les revendications 1 à 6, destinés à combattre les maladies de la circulation sanguine.

9. Médicaments contenant comme substance active un ou plusieurs composés selon les revendications 1 à 6.

10. Procédé de préparation de médicaments selon la revendication 9, caractérisé en ce qu'on mélange les composés selon les revendications 1 à 6 avec des supports, excipients ou solvants inertes, pharmaceutiquement convenables et non toxiques.

11. Utilisation des composés selon les revendications 1 à 6 dans la fabrication de médicaments agissant sur la circulation du sang.